# EUROPÄISCHE PATENTSCHRIFT

(11) **EP 3 328 871 B1**
(45) Veröffentlichungstag und Bekanntmachung des Hinweises auf die Patenterteilung: **10.07.2019**
(21) Anmeldenummer: 16745109.5
(22) Anmeldetag: 29.07.2016
(51) Int. Cl.: C07F 9/38, C07F 9/40, A61K 6/033

(54) **VERNETZENDE MONOMERE MIT MINDESTENS EINEM SCHWEFELATOM**
CROSSLINKING MONOMERS WITH AT LEAST ONE ATOM OF SULPHUR
MONOMÈRES DE RÉTICULATION COMPORTANT AU MOINS UN ATOME DE SOUFRE

(30) Priorität: 31.07.2015 DE 102015112602
(43) Veröffentlichungstag der Anmeldung: 06.06.2018
(73) Patentinhaber: Kulzer GmbH, 63450 Hanau (DE)
(72) Erfinder: UTTERODT, Andreas, 61267 Neu Anspach (DE); RITTER, Helmut, 42111 Wuppertal (DE); TABATABEI, Monir, 40225 Düsseldorf (DE); KEMNITZ, Melanie Nadine, 65549 Limburg (DE)
(74) Vertreter: Schultheiss & Sterzel Patentanwälte PartG mbB
(86) Internationale Anmeldenummer: PCT/EP2016/068155
(87) Internationale Veröffentlichungsnummer: WO 2017/021297

(56) Entgegenhaltungen:
- JP-A- 2008 250 226
- KEMNITZ, MELANIE ET AL: "Phosphonate-Containing Polymeric Networks: Swelling in Water Controlled by Metal Ions", MACROMOLECULAR CHEMISTRY AND PHYSICS , 216(3), 314-320 CODEN: MCHPES; ISSN: 1022-1352, 2015, XP002762177,
- CATEL, YOHANN ET AL: "Synthesis and photopolymerization of phosphonic acid monomers for applications in compomer materials", JOURNAL OF APPLIED POLYMER SCIENCE , 117(5), 2676-2687 CODEN: JAPNAB; ISSN: 0021-8995, 2010, XP002762178,
- QUITTMANN, ULRICH ET AL: "Synthesis of a new phosphonated dimethacrylate: photocuring kinetics in homo- and copolymerization, determination of thermal and flame-retardant properties", MACROMOLECULAR CHEMISTRY AND PHYSICS , 202(5), 628-635 CODEN: MCHPES; ISSN: 1022-1352, 2001, XP002762179,
- SIBOLD, NATHALIE ET AL: "Synthesis and characterization of (co)polymers containing a phosphonate function for use in dental composites", POLYMER , 43(26), 7257-7267 CODEN: POLMAG; ISSN: 0032-3861, 2002, XP002762180,

## Beschreibung

Die Erfindung betrifft eine Härtbare Zusammensetzung zur Verwendung in der Dentalmedizin, umfassend mindestens ein vernetzendes Monomer mit mindestens einem Schwefelatom.

Polymerisierbare Phosphonsäuren sind vor allem als Co-monomere von polymerchemischer und technischer Bedeutung und gestatten die Herstellung von organischen Polymeren mit besserer thermischer Stabilität, verbesserten Hafteigenschaften, verminderter Entflammbarkeit und verbesserter Löslichkeit in polaren Lösungsmitteln. In diesem Zusammenhang sind zahlreiche monomere Phosphonsäuren mit polymerisierbaren Vinyl-, Dienyl-, Allyl- oder Styrylgruppen synthetisiert und polymerisiert worden (vgl. Houben-Weyl, Methoden der Organischen Chemie, Band E 20 (2. eil), G. Thieme Verlag, Stuttgart-New York 1987, 1300 ff.). Polymerisierbare Phosphonsäuren sind auch als Komponente von Dentaladhäsiven bekannt (vgl. N. Moszner, U. Salz, J. Zimmermann, Dental Materials 21 (2005) 895-910) und Gegenstand zahlreicher Patente und Patentanmeldungen, wie z.B. DE 100 18 968 C1, DE 102 34 326 B3, DE 199 18 974 A1, EP 1 057 468 A1 und EP 1 169 996 A1. Verbindungen, die in Zusammensetzungen für die Zahnmedizin verwendet werden können, sind offenbart in Polymer 43 (2002) 7257-7267.

Die Druckschrift WO 2013/083734 A1 beschreibt polymerisierbare Bisphosphonsäuren, die ähnlich den zuvor genannten Monomeren in eine Polymermatrix eingebunden werden können. Schwefelhaltige Monomere werden in WO 2013/083734 A1 nicht explizit genannt. In einer sehr allgemeinen, nicht als bevorzugt ausgeführten Ausgestaltung können Schwefelatome, beispielsweise als Thioethergruppe -S- in Ketten vorhanden sein, die mindestens eine polymerisierbare Gruppe mit einem (m+2)-fach substituierten aliphatischen C₁-C₈-Rest verbindet, der in WO 2013/083734 A1 mit dem Symbol A bezeichnet wird. Der Rest A umfasst keine Schwefelatome und ist mit zwei Phosphonsäuregruppen verbunden. Zwischen den zwei Phosphonsäuregruppen können daher in Monomeren gemäß WO 2013/083734 A1 keine Schwefelatome angeordnet sein. Weiterhin stellt die Gruppe A eine Verzweigungsstelle dar, da alle polymerisierbaren Gruppen an die Gruppe A binden. Eine Ausgestaltung, bei der ein Schwefelatom zwischen einem Phosphoratom und einer Verzweigungsstelle angeordnet ist, findet sich daher nicht.

Ferner finden sich keine konkreten Hinweise auf Schwefelatom-enthaltende Monomere. Die genannte Ausgestaltung ist vielmehr ausgewählt aus einer langen Liste von Gruppen, die in einer Kette vorhanden sein können, welche eine polymerisierbare Gruppe mit dem Rest A verbindet.

Die zuvor genannten Monomere führen zu brauchbaren Dentalmaterialien. Jedoch besteht das dauerhafte Bedürfnis die Eigenschaften dieser Materialien zu verbessern. Insbesondere sollte die Haftung dieser Materialien auf verschiedenen Substraten, die in der Dentalmedizin von Bedeutung sind, verbessert werden. Ferner sollte die Formulierung von Zusammensetzungen zur Herstellung dieser Dentalmaterialien vereinfacht werden. Eine weitere Aufgabe kann darin gesehen werden, die Handhabung der dentalmedizinisch einsetzbaren Formulierungen einfacher zu gestalten. Darüber hinaus sollte die Härtung von polymerisierbaren Zusammensetzungen verbessert werden. Weiterhin sollten die mechanischen Eigenschaften von polymerisierten Dentalmaterialien gesteigert werden.

Gelöst werden diese sowie weitere nicht explizit genannte Aufgaben, die jedoch aus den hierin einleitend diskutierten Zusammenhängen ohne Weiteres ableitbar oder erschließbar sind, durch ein Monomer umfassend mindestens ein Schwefelatom mit allen Merkmalen des Patentanspruchs 1. Zweckmäßige Abwandlungen der erfindungsgemäßen Monomere werden in den auf Anspruch 1 rückbezogenen abhängigen Ansprüchen unter Schutz gestellt.

Gegenstand der vorliegenden Erfindung ist daher eine Härtbare Zusammensetzung zur Verwendung in der Dentalmedizin, umfassend mindestens ein vernetzendes Monomer mit mindestens einem Schwefelatom, darstellbar durch eine Struktur der Formel (I) 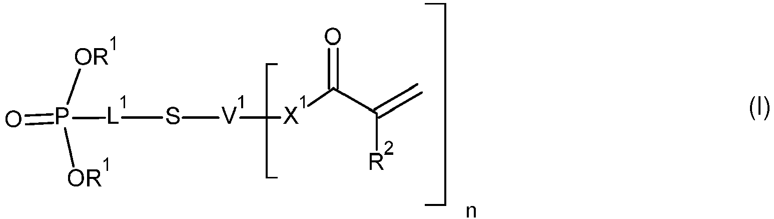 worin die Symbole folgende Bedeutung aufweisen:
- L1: ist eine lineare, verzweigte oder cyclische Alkylengruppe mit 2 bis 10 Kohlenstoffatomen ist, in der ein oder mehrere nicht benachbarte CH₂ -Gruppen durch -R³C=CR³-, -C=C-, -(C=O)-, -(C=S)-, -(C=NR³)-, -C(=O)O-, -C(=0)NR³-,-NR³ , P(=O)(R³), -[CH(CₓH₂ₓ)P(=O)(OR¹)₂]-, -O-, -S-, -(SO)- oder (-SO₂)- ersetzt sein können, wobei x eine ganze Zahl im Bereich von 0 bis 6 ist, wobei das Phosphoratom der Gruppe -P=O(OR¹)₂ direkt mit einem Kohlestoffatom verbunden ist;
- V¹: ist eine gesättigte oder ungesättigte, aliphatische oder heteroaliphatische Gruppe mit 2 bis 50 Kohlenstoffatomen; eine aromatische oder heteroaromatische Gruppe mit 5 bis 50 Kohlenstoffatomen oder eine Gruppe mit 6 bis 50 Kohlenstoffatomen, die mindestens einen gesättigten oder ungesättigten, aliphatischen und/oder heteroaliphatischen Rest und mindestens einen aromatischen und/oder heteroaromatischen Rest umfasst;
- X¹: ist bei jedem Auftreten gleich oder verschieden O oder NR³;
- R¹: ist H, Methyl, Ethyl, Propyl oder Butyl;
- R²: ist H oder ein Alkylrest mit 1 bis 4 Kohlenstoffatomen;
- R³: ist H oder ein aliphatische, heteroaliphatische, aromatische oder heteroaromatische Gruppe mit 1 bis 6 Kohlenstoffatomen und
- n: ist 2, 3, 4, 5 oder 6,
welches dadurch gekennzeichnet ist, dass
das Schwefelatom in der Struktur gemäß Formel (I) über höchstens 12 Bindungen von der Gruppe X¹ getrennt ist, die von dem Schwefelatom über die geringste Anzahl an Bindungen entfernt ist, wobei Doppelbindungen oder Dreifachbindungen als Einfachbindung gezählt werden.

Die erfindungsgemäßen Härtbare Zusammensetzung umfassend mindestens ein vernetzendes Monomer mit mindestens einem Schwefelatom sind insbesondere für Anwendungen in der Zahnmedizin geeignet und weisen ein gutes Haftvermögen auf, insbesondere gegenüber Zahnhartsubstanz und/oder Metallen, die in der Zahnmedizin eingesetzt werden.

Die erfindungsgemäßen Härtbare Zusammensetzung umfassend mindestens ein vernetzendes Monomer mit mindestens einem Schwefelatom weisen bevorzugt im ausgehärteten Zustand in Erzeugnissen ein sehr gutes Haftvermögen auf festen Oberflächen auf, vorzugsweise gegenüber Dentin, dabei mit einer Haftkraft von bevorzugt zumindest 12,5 MPa.

Die erfindungsgemäßen Härtbare Zusammensetzung umfassend mindestens ein vernetzendes Monomer mit mindestens einem Schwefelatom sind als Additive für und als Bestandteil von haftverbessernde(n) Adhäsive(n) insbesondere für mineralische Untergründe und besonders Zahnhartmaterialien geeignet. Sie ermöglichen auch die Anwendung im Non-Etch Verfahren, da die erfindungsgemäßen Verbindungen, auch in Kombination mit einem oder mehreren weiteren Monomeren, offenbar die für das Bondingprozedere notwendige Oberflächenmodifizierung bewirken.

Weiterhin zeigen die erfindungsgemäßen Härtbare Zusammensetzung umfassend mindestens Monomere gemäß Formel (I) eine haftverbessernde Wirkung als Bestandteil von dentalen Füllungskompositen und Befestigungszementen, von Unterfüllungsmaterialien, von Flow-Materialien, von Fissurenversieglern, von Lacken, von Wurzelkanalmaterialien, von Stumpfaufbaumaterialien und von provisorischen Restaurationsmaterialien (insbesondere Inlays, Onlays, Kronen, Brücken und/oder Befestigungsmaterialien).

Hierbei führen die erfindungsgemäßen Härtbare Zusammensetzung umfassend mindestens Monomere mit mindestens einem Schwefelatom bevorzugt im ausgehärteten Zustand zu Erzeugnissen mit ausgezeichneten mechanischen Eigenschaften und einer hohen Beständigkeit. Hierzu gehören eine hohe Abriebfestigkeit (Verschleißfestigkeit) und eine hohe Haltbarkeit bei Belastung (Biegefestigkeit). Ferner führen die Monomere gemäß der vorliegenden Erfindung vorzugsweise zu einer geringen oder verminderten Wasseraufnahme oder Löslichkeit.

Darüber hinaus lassen sich die erfindungsgemäßen Härtbare Zusammensetzung umfassend mindestens Monomere mit mindestens einem Schwefelatom sehr gut verarbeiten, wobei sich dies in einer guten Löslichkeit in verschiedenen Lösungsmitteln und einer relativ guten und schnellen Polymerisation zeigt.

Die genannten Vorteile können vollständig oder teilweise verwirklicht sein. Im Allgemeinen zeichnen sich die Monomere durch eine verbesserte Haftung auf verschiedenen Materialien aus.

Der Begriff "vernetzendes Monomer" bezeichnet ein Monomer, welches mindestens zwei radikalisch polymerisierbare Gruppen, vorzugsweise (Meth)acrylgruppen aufweist. (Meth)acrylgruppen sind Gruppen die sich beispielsweise von Acrylsäure, Methacrylsäure, Acrylamid oder Methacrylamid ableiten.

Benachbarte Kohlenstoffatome im Sinne der vorliegenden Erfindung sind Kohlenstoffatome, die direkt miteinander verknüpft sind. Weiterhin bedeutet "benachbarte Reste" in der Definition der Reste, dass diese Reste an dasselbe Kohlenstoffatom oder an benachbarte Kohlenstoffatome gebunden sind. Diese Definitionen gelten entsprechend unter anderem für die Begriffe "benachbarte Gruppen" und "benachbarte Substituenten". Der Begriff "nicht benachbarte" Kohlenstoffatome ergibt sich komplementär aus dieser Definition.

Der Begriff lineare, verzweigte oder cyclische, gesättigte oder ungesättigte, aliphatische oder heteroaliphatische Gruppe mit 2 bis 10 Kohlenstoffatomen ist in der Fachwelt bekannt und bezeichnet eine Gruppe mit 2 bis 10 Kohlenstoffatomen, die keine aromatische oder heteroaromatische Gruppe zwischen den mindestens zwei Bindungsstellen der jeweiligen Gruppe umfasst und bevorzugt keine aromatische oder heteroaromatische Gruppe aufweist. So weist beispielsweise die Gruppe L¹ mindestens eine Bindungsstelle zum Phosphoratom und mindestens eine Bindungsstelle zum Schwefelatom auf. Der Begriff "keine aromatische oder heteroaromatische Gruppe zwischen den mindestens zwei Bindungsstellen" bedeutet, dass keine aromatische oder heteroaromatische Gruppe an den Bindungen zwischen dem Phosphoratom und dem Schwefelatom teilnimmt, wohl aber als Seitengruppe oder Substituent vorhanden sein kann.

Zu den gesättigten aliphatischen Gruppen, die beispielsweise als Gruppe L¹ in Formel (I) geeignet sind, gehören unter anderem Ethylen, 1,2- und 1,3-Propylen, 1,2-, 1,3- und 1,4-Butylen, Pentylen, Hexylen, Heptylen, Octylen, Nonylen, Decylen, Undecylen, Dodecylen und Tetradecylen.

Ungesättigt aliphatischen Gruppen, die beispielsweise als Gruppe L¹ in Formel (I) geeignet sind, unterscheiden sich von den zuvor genannten Gruppen durch das Vorhandensein mindestens einer C-C-Doppel- oder Dreifachbindung. Hierzu gehören unter anderem Ethenylen, 1,2- und 1,3-Propenylen, 1,2-, 1,3- und 1,4-Butenylen, Pentenylen, Hexenylen, Heptenylen, Octenylen, Nonenylen, Decenylen, Undecenylen, Dodecenylen und Tetradecenylen.

Heteroaliphatische Gruppen weisen neben Kohlenstoff- und Wasserstoffatomen Heteroatome, wie Stickstoff-, Sauerstoff-, Phosphor-, Silicium- und Schwefelatome auf. Bevorzugte heteroaliphatische Gruppen, die gesättigt oder ungesättigt sein können, leiten sich unter anderem von den zuvor genannten gesättigten und ungesättigten aliphatischen Gruppen ab, wobei ein oder mehrere nicht benachbarte CH₂-Gruppen durch -(C=O)-, -(C=S)-, -(C=NR³)-, -C(=O)O-, -C(=O)NR³-, -NR³-, P(=O)(R³), -[CH(CₓH₂ₓ)P(=O)(OR¹)₂]-, -O-, -S-, -(SO)- oder (-SO₂)- ersetzt sind, wobei R³ H oder eine aliphatische, heteroaliphatische, aromatische oder heteroaromatische Gruppe mit 1 bis 6 Kohlenstoffatomen und x eine ganze Zahl im Bereich von 0 bis 6, vorzugsweise 1 bis 5 ist.

In einer besonderen Ausgestaltung kann das Symbol L¹ eine lineare, verzweigte oder cyclische Alkylengruppe mit 2 bis 10 Kohlenstoffatomen darstellen, in der ein oder mehrere nicht benachbarte CH₂-Gruppen durch -R³C=CR³-, -C=C-, -(C=O)-, -(C=S)-, -(C=NR³),-, -C(=O)O-, -C(=O)NR³-, -NR³-, P(=O)(R³), -[CH(CₓH₂ₓ)P(=O)(OR¹)₂]-, O-, -S-, -(SO)- oder (-SO₂)- ersetzt sein können, wobei R³ die zuvor für Formel (I) genannte Bedeutung aufweist und x eine ganze Zahl im Bereich von 0 bis 6, vorzugsweise von 1 bis 5 ist. Falls die Gruppe L¹ ein Phosphoratom aufweist, ist dieses Phosphoratom vorzugsweise über mindestens 4, besonders bevorzugt über mindestens 6 Bindungen von dem in Formel (I) dargestellten Phosphoratom entfernt. In einer weiteren Ausgestaltung kann das zweite Phosphoratom näher zum Schwefelatom angeordnet sein als das erste Phosphoratom. Näher angeordnet bedeutet, dass die Anzahl der Atome, die zwischen dem Schwefelatom und den Phosphoratomen vorhanden sind, unterschiedlich ist.

Besonders bevorzugt ist L¹ eine lineare, verzweigte oder cyclische Alkylengruppe mit 2 bis 10, vorzugsweise 3 bis 5 Kohlenstoffatomen, die keine Heteroatome aufweist. Besonders bevorzugt stellt die Gruppe L¹ einen gesättigten Kohlenwasserstoffrest mit 2 bis 6, vorzugsweise 3 bis 5 Kohlenstoffatome dar, die keine Heteroatome umfasst.

Das Phosphoratom in Formel (I) ist direkt mit einem Kohlestoffatom der Gruppe L¹ verbunden, so dass die das Phosphoratom enthaltende Gruppe als Phosphonsäure- oder Phosphonsäureester-Gruppe aufgefasst werden kann.

Vorzugsweise kann die Härtbare Zusammensetzung umfassend mindestens ein vernetzendes Monomer mit mindestens einem Schwefelatom durch eine Struktur der Formel (Ia) darstellt werden 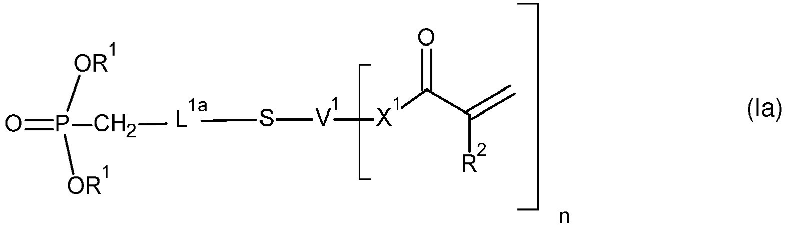 worin die Symbole n, V¹, X¹, R¹ und R² die zuvor, insbesondere für Formel (I) genannte Bedeutung aufweisen und
- L^{1a}: eine lineare, verzweigte oder cyclische, gesättigte oder ungesättigte, aliphatische oder heteroaliphatische Gruppe mit 2 bis 9 Kohlenstoffatomen; eine aromatische oder heteroaromatische Gruppe mit 4 bis 12, vorzugsweise 5 bis 10 Kohlenstoffatomen oder eine Gruppe mit 6 bis 13 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist.

Bevorzugte Ausgestaltungen der Gruppe L^{1a} ergeben sich aus den zuvor dargestellten speziellen Ausführungsformen der Gruppe L¹, wobei jedoch die CH₂-Gruppe zu berücksichtigen ist.

Das Symbol R¹ in Formel (I) oder (la) ist H, Methyl, Ethyl, Propyl oder Butyl; R¹ besonders bevorzugt für Wasserstoff oder Ethyl.

Die Gruppe V¹ ist eine gesättigte oder ungesättigte, aliphatische oder heteroaliphatische Gruppe mit 2 bis 50, vorzugsweise 3 bis 30 Kohlenstoffatomen; eine aromatische oder heteroaromatische Gruppe mit 5 bis 50, vorzugsweise 6 bis 30 Kohlenstoffatomen oder eine Gruppe mit 6 bis 50, vorzugsweise 6 bis 30 Kohlenstoffatomen, die mindestens einen gesättigten oder ungesättigten, aliphatischen und/oder heteroaliphatischen Rest und mindestens einen aromatischen und/oder heteroaromatischen Rest umfasst. Hierbei gelten die zuvor dargelegten Definitionen entsprechend, wobei die Anzahl der Kohlenstoffatome jedoch höher sein kann. Die Gruppe V¹ weist mindestens drei Bindungsstellen auf. Die genaue Anzahl der Bindungsstellen errechnet sich aus der Anzahl der polymerisierbaren Acrylreste n, die in Formel (I) oder (la) angegeben ist. Der Rest V1 ist dementsprechend (n+1)-wertig.

Bevorzugt stellt die Gruppe V¹ eine gesättigte oder ungesättigte, aliphatische oder heteroaliphatische Gruppe mit 2 bis 50, vorzugsweise 3 bis 30 Kohlenstoffatomen oder eine Gruppe mit 6 bis 50, vorzugsweise 6 bis 30 Kohlenstoffatomen dar, die mindestens einen gesättigten oder ungesättigten, aliphatischen und/oder heteroaliphatischen Rest und mindestens einen aromatischen und/oder heteroaromatischen Rest umfasst. Bevorzugt ist hierbei, dass das Schwefelatom in Formel (I) und die Gruppe X¹ in Formel (I) jeweils über ein aliphatisches Kohlenstoffatom der Gruppe V¹ gebunden ist, vorzugsweise eine Gruppe -CH₂- oder -CHR³-, wobei R³ die für Formel (I) genannte Bedeutung aufweist.

Der Index n steht für die Anzahl der Acrylgruppen, wobei dieser Index eine ganze Zahl im Bereich von 2 bis 6, vorzugsweise 2 bis 4 und besonders bevorzugt 2 oder 3 ist.

Das Symbol X¹ in Formel (I) oder (la) ist bei jedem Auftreten gleich oder verschieden O oder NR³, wobei R³ H oder eine aliphatische, heteroaliphatische, aromatische oder heteroaromatische Gruppe mit 1 bis 6 Kohlenstoffatomen ist. Hierbei steht Symbol X¹ in Formel (I) oder (la) vorzugsweise für O. Bevorzugt steht R³ für Wasserstoff, Methyl, Ethyl, Propyl, Butyl oder Phenyl. Gemäß einer besonderen Ausgestaltung kann mindestens ein X¹ für NR³, und mindestens ein X¹ für O stehen.

Das Symbol R² ist H oder ein Alkylrest mit 1 bis 4 Kohlenstoffatomen, vorzugsweise H oder Methyl und besonders bevorzugt Methyl.

Das erfindungsgemäße Härtbare Zusammensetzung umfassend mindestens ein vernetzendes Monomer mit mindestens einem Schwefelatom zeichnet sich dadurch aus, dass das Schwefelatom in der Struktur gemäß Formel (I) oder (la) über höchstens 12 Bindungen von der Gruppe X¹ getrennt ist, die von dem Schwefelatom über die geringste Anzahl an Bindungen entfernt ist, wobei Doppelbindungen oder Dreifachbindungen als Einfachbindung gezählt werden. Vorzugsweise ist das Schwefelatom in der Struktur gemäß Formel (I) oder (la) über höchstens 10, besonders bevorzugt über höchstens 8 Bindungen von der Gruppe X¹ getrennt, die von dem Schwefelatom über die geringste Anzahl an Bindungen entfernt ist, wobei Doppelbindungen oder Dreifachbindungen als Einfachbindung gezählt werden.

Demgemäß befinden sich zwischen dem Schwefelatom und dem Sauerstoff- oder Stickstoffatom der Gruppe X¹ höchstens 11, vorzugsweise höchstens 9 und speziell bevorzugt höchstens 7 kovalent gebundene Atome. Dieser Wert gilt für das Sauerstoff- oder Stickstoffatom der Gruppe X¹, welches die geringste Entfernung zum Schwefelatom in der Struktur gemäß Formel (I) oder (la) aufweist.

In einer weiteren Ausgestaltung kann vorgesehen sein, dass das Schwefelatom in der Struktur gemäß Formel (I) oder (la) über höchstens 12 Bindungen von der Gruppe X¹ getrennt ist, die von dem Schwefelatom über die höchste Anzahl an Bindungen entfernt ist, wobei Doppelbindungen oder Dreifachbindungen als Einfachbindung gezählt werden. Vorzugsweise ist das Schwefelatom in der Struktur gemäß Formel (I) oder (la) über höchstens 10, besonders bevorzugt über höchstens 8 Bindungen von der Gruppe X¹ getrennt, die von dem Schwefelatom über die höchste Anzahl an Bindungen entfernt ist, wobei Doppelbindungen oder Dreifachbindungen als Einfachbindung gezählt werden.

Ferner kann vorgesehen sein, dass das Phosphoratom in der Struktur gemäß Formel (I) oder (la) über höchstens 20 Bindungen von der Gruppe X¹ getrennt ist, die von dem Phosphoratom über die höchste Anzahl an Bindungen entfernt ist, wobei Doppelbindungen oder Dreifachbindungen als Einfachbindung gezählt werden. Vorzugsweise ist das Phosphoratom in der Struktur gemäß Formel (I) oder (la) über höchstens 18, besonders bevorzugt über höchstens 16 Bindungen von der Gruppe X¹ getrennt, die von dem Schwefelatom über die höchste Anzahl an Bindungen entfernt ist, wobei Doppelbindungen oder Dreifachbindungen als Einfachbindung gezählt werden.

Ferner kann vorgesehen sein, dass das Phosphoratom in der Struktur gemäß Formel (I) oder (la) über mindestens 8 Bindungen von der Gruppe X¹ getrennt ist, die von dem Phosphoratom über die geringste Anzahl an Bindungen entfernt ist, wobei Doppelbindungen oder Dreifachbindungen als Einfachbindung gezählt werden. Vorzugsweise ist das Phosphoratom in der Struktur gemäß Formel (I) oder (la) über mindestens 10, besonders bevorzugt über mindestens 11 Bindungen von der Gruppe X¹ getrennt, die von dem Schwefelatom über die geringste Anzahl an Bindungen entfernt ist, wobei Doppelbindungen oder Dreifachbindungen als Einfachbindung gezählt werden.

Erfindungsgemäße Härtbare Zusammensetzung umfassend mindestens ein vernetzendes Monomer gemäß Formel (I) oder (la), in der das Phosphor- oder das Schwefelatom über eine geringe Anzahl an Bindungen von der Gruppe X¹ getrennt ist, führen zu verbesserten Polymereigenschaften von härtbaren Zusammensetzungen. Ferner zeigen diese Verbindungen eine relativ geringe Viskosität.

Der Ausdruck "zwischen den Atomen" oder "zwischen den Bindungsstellen" bedeutet, dass die Atome oder Bindungen in einer Hauptkette vorhanden sind, die die beiden Bindungsstellen oder die beiden Atome verbinden. Der Begriff "Hauptkette" ist als Abgrenzung zu einer "Seitenkette" oder einem Substituenten zu sehen, der von der Hauptkette abzweigt. Vereinfacht gesagt ändert sich die Zahl der "zwischen den Atomen" oder "zwischen den Bindungsstellen" angeordneten Bindungen oder Atome nicht, falls eine Seitengruppe oder ein Substituent durch ein Wasserstoffatom ersetzt wird. Falls in einer Hauptkette ein Atom durch ein oder zwei Wasserstoffatome ersetzt wird, ändert sich diese Zahl.

Ferner kann vorgesehen sein, dass die Gruppe V¹ in Formel (I) oder (la) mindestens einen aromatischen und/oder heteroaromatischen Rest aufweist, vorzugsweise mindestens einen aromatischen Rest. Dieser aromatische und/oder heteroaromatische Rest kann Teil einer linearen, verzweigten oder cyclischen, gesättigten oder ungesättigten, aliphatischen oder heteroaliphatischen Gruppe sein, wobei der Rest in diesem Fall beispielsweise als Substituent einer CH₂-Gruppe oder einer Gruppe der Formeln -(C=NR³)-, -C(=O)NR³-, -NR³-, P(=O)(R³) sein kann. Vorzugsweise kann der aromatische und/oder heteroaromatische Rest der Gruppe V¹ ein monocyclischer Rest sein, vorzugsweise ein Phenyl- oder Phenylen-Rest, der gegebenenfalls durch ein oder mehrere Gruppen R³ substituiert sein kann, wobei R³ die zuvor im Zusammenhang mit Formel (I) genannte Bedeutung aufweist.

Erfindungsgemäße Härtbare Zusammensetzung umfassend mindestens Monomere gemäß Formel (I) oder (la), in der die Gruppe V¹ mindestens einen aromatischen und/oder heteroaromatischen Rest aufweist, führen zu verbesserten mechanischen Eigenschaften von gehärteten Zusammensetzungen.

Bevorzugt kann vorgesehen sein, dass ein Monomer gemäß Formel (I) oder (la) insgesamt 1, 2 oder 3 Schwefelatome, besonders bevorzugt 1 oder 2 Schwefelatome und speziell bevorzugt genau ein Schwefelatom aufweist, wobei diese Zahl das In Formel (I) oder (la) dargestellte Schwefelatom einschließt. Falls mehr als ein Schwefelatom enthalten ist, kann dieses in der Gruppe L¹ und/oder der Gruppe V¹ enthalten sein.

Bevorzugt kann vorgesehen sein, dass ein Monomer gemäß Formel (I) oder (la) insgesamt 1, 2 oder 3 Phosphoratome, besonders bevorzugt 1 oder 2 Phosphoratome und speziell bevorzugt genau ein Phosphoratom aufweist, wobei diese Zahl das In Formel (I) oder (la) dargestellte Phosphoratom einschließt. Falls mehr als ein Phosphoratom enthalten ist, kann dieses in der Gruppe L¹ und/oder der Gruppe V¹ enthalten sein.

In einer bevorzugten Ausgestaltung kann das erfindungsgemäße Härtbare Zusammensetzung umfassend mindestens ein vernetzendes Monomer mit mindestens einem Schwefelatom durch eine Struktur (II) darstellbar sein 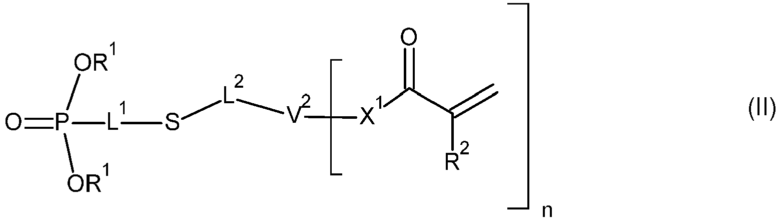 worin die Symbole n, L¹, X¹, R¹ und R² die zuvor insbesondere für Formel (I) dargelegte Bedeutung aufweisen und
- V²: eine gesättigte oder ungesättigte, aliphatische und/oder heteroaliphatische Gruppe mit 2 bis 10, vorzugsweise 3 bis 6 Kohlenstoffatomen; eine aromatische und/oder heteroaromatische Gruppe mit 4 bis 12, vorzugsweise 5 bis 10 Kohlenstoffatomen oder eine Gruppe mit 6 bis 14 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist; vorzugsweise ein Kohlenwasserstoffrest mit 3 bis 5 Kohlenstoffatomen; und
- L²: eine lineare, verzweigte oder cyclische, gesättigte oder ungesättigte, aliphatische und/oder heteroaliphatische Ether- oder Estergruppe mit 2 bis 30 Kohlenstoffatomen; eine aromatische und/oder heteroaromatische Ether- oder Estergruppe mit 5 bis 30 Kohlenstoffatomen oder eine Ether- oder Estergruppe mit 6 bis 30 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist.

Der Begriff Ethergruppe ist in der Fachwelt bekannt, wobei eine Ethergruppe mindestens ein Sauerstoffatom aufweist, welches mit zwei Kohlenstoffatomen verbunden ist. Hierbei kann das mit dem Sauerstoffatom verbundene Kohlenstoffatom jeweils Bestandteil einer linearen, verzweigten oder cyclischen, gesättigten oder ungesättigten, aliphatischen oder heteroaliphatischen Gruppe; einer aromatische oder heteroaromatische Gruppe oder einer Gruppe sein, die mindestens einen gesättigten oder ungesättigten, aliphatischen und/oder heteroaliphatischen Rest und mindestens einen aromatischen und/oder heteroaromatischen Rest umfasst. Die Anzahl an Kohlenstoffatomen ergibt sich aus der Gesamtzahl beider an das Sauerstoffatom der Ethergruppe gebundenen kohlenstoffhaltigen Reste. Zu berücksichtigen ist jedoch, dass eines der Kohlenstoffatome, an die das Sauerstoffatom bindet, Bestandteil einer benachbarten Gruppe, beispielsweise der Gruppe V² oder der nachfolgend in Formel (III) beschriebenen Gruppe L³ sein kann. Der Begriff Estergruppe ist in der Fachwelt bekannt, wobei eine Estergruppe mindestens eine Gruppe der Form -C(=O)O- aufweist, welche mit zwei Kohlenstoffatomen verbunden ist. Hierbei kann das mit der -C(=O)O- Gruppe verbundene Kohlenstoffatom jeweils Bestandteil einer linearen, verzweigten oder cyclischen, gesättigten oder ungesättigten, aliphatischen oder heteroaliphatischen Gruppe; einer aromatische oder heteroaromatische Gruppe oder einer Gruppe sein, die mindestens einen gesättigten oder ungesättigten, aliphatischen und/oder heteroaliphatischen Rest und mindestens einen aromatischen und/oder heteroaromatischen Rest umfasst. Die Anzahl an Kohlenstoffatomen ergibt sich aus der Gesamtzahl beider an die -C(=O)O- Gruppe gebundenen kohlenstoffhaltigen Reste. Zu berücksichtigen ist jedoch, dass eines der Kohlenstoffatome, an die die - C(=O)O- Gruppe bindet, Bestandteil einer benachbarten Gruppe, beispielsweise der Gruppe V² oder der nachfolgend in Formel (III) beschriebenen Gruppe L³ sein kann. Bevorzugt kann vorgesehen sein, dass ein Monomer gemäß Formel (II) insgesamt 1, 2 oder 3 Schwefelatome, besonders bevorzugt 1 oder 2 Schwefelatome und speziell bevorzugt genau ein Schwefelatom aufweist, wobei diese Zahl das In Formel (II) dargestellte Schwefelatom einschließt. Falls mehr als ein Schwefelatom enthalten ist, kann dieses in der Gruppe L¹, L² und/oder V² enthalten sein, vorzugsweise in Gruppe L¹ und/oder L².

Bevorzugt kann vorgesehen sein, dass ein Monomer gemäß Formel (II) insgesamt 1, 2 oder 3 Phosphoratome, besonders bevorzugt 1 oder 2 Phosphoratome und speziell bevorzugt genau ein Phosphoratom aufweist, wobei diese Zahl das In Formel (II) dargestellte Phosphoratom einschließt. Falls mehr als ein Phosphoratom enthalten ist, kann dieses in der Gruppe L¹, L² und/oder V² enthalten sein, vorzugsweise in Gruppe L¹ und/oder L².

In einer bevorzugten Ausgestaltung kann das erfindungsgemäße Härtbare Zusammensetzung umfassend mindestens ein vernetzendes Monomer mit mindestens einem Schwefelatom durch eine Struktur (IIa) darstellbar sein 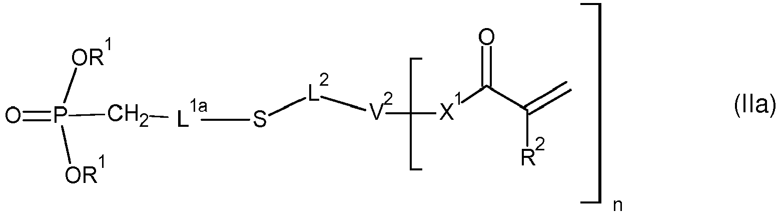 worin die Symbole n, L^{1a}, X¹, R¹ und R² die zuvor insbesondere für Formel (la) und die Gruppen L² und V² die zuvor insbesondere für Formel (II) genannte Bedeutung aufweisen.

Bevorzugt kann vorgesehen sein, dass die Gruppe V² ausgewählt ist aus folgenden Strukturen (V²-1), (V²-2) und/oder (V²-3) 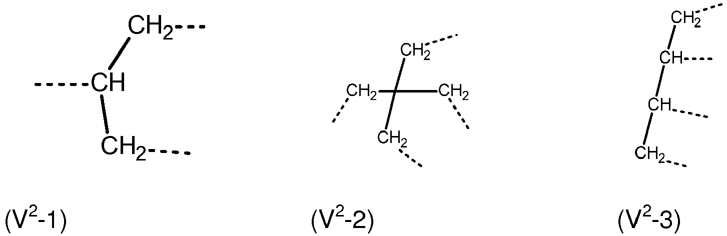 wobei die gestrichelten Linien die Bindungen an die Gruppen L² oder X¹ darstellen.

Weiterhin kann vorgesehen sein, dass die Gruppe L² durch bevorzugt eine Struktur der Formel (L²-1) darstellbar ist 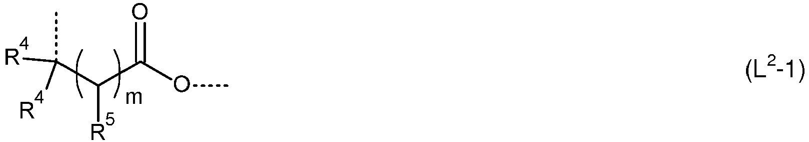 worin R⁴ bei jedem Auftreten H, eine Alkylgruppe mit 1 bis 10 Kohlenstoffatomen oder eine aromatische Gruppe mit 6 bis 8 Kohlenstoffatomen, vorzugsweise H oder eine Phenylgruppe; R⁵ bei jedem Auftreten unabhängig H oder eine Alkylgruppe mit 1 bis 10 Kohlenstoffatomen, vorzugsweise H; der Index m eine ganze Zahl im Bereich von 0 bis 8, vorzugsweise 0 bis 7 und besonders bevorzugt 0 bis 5 ist und die gestrichelten Linien jeweils die Bindung an das Schwefelatom oder die Gruppe V² darstellen.

Ferner kann vorgesehen sein, dass das Monomer mit mindestens einem Schwefelatom bevorzugt durch die Struktur (III) darstellbar ist 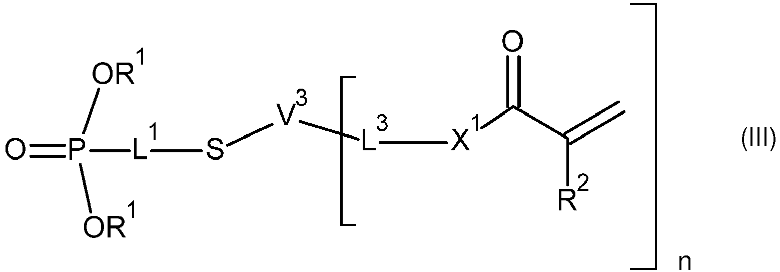 worin die Symbole n, L¹, X¹, R¹ und R² die zuvor, insbesondere für Formel (I) dargelegte Bedeutung aufweisen,
- V³: eine gesättigte oder ungesättigte, aliphatische und/oder heteroaliphatische Gruppe mit 2 bis 10, vorzugsweise 3 bis 6 Kohlenstoffatomen; eine aromatische und/oder heteroaromatische Gruppe mit 4 bis 12, vorzugsweise 5 bis 10 Kohlenstoffatomen oder eine Gruppe mit 6 bis 14 Kohlenstoffatomen ist, die mindestens einen gesättigten oder ungesättigten, aliphatischen und/oder heteroaliphatischen Rest und mindestens einen aromatischen und/oder heteroaromatischen Rest umfasst; vorzugsweise eine aliphatische Gruppe mit 2 bis 10, vorzugsweise 3 bis 6 Kohlenstoffatomen, in der ein oder mehrere nicht benachbarte CH₂-Gruppen durch -R³C=CR³-, -C≡C-, -(C=O)-, -(C=S)-,-(C=NR³),-, -C(=O)O-, -C(=O)NR³-, -NR³-, P(=O)(R³), -[CH(CₓH₂ₓ)P(=O)(OR¹)₂]-, -O-, -S-, -(SO)- oder (-SO₂)- ersetzt sein können, wobei R³ die zuvor für Formel (I) genannte Bedeutung aufweist und x eine ganze Zahl im Bereich von 0 bis 6, vorzugsweise 1 bis 5 ist; und
- L³: bei jedem Auftreten unabhängig eine lineare, verzweigte oder cyclische, gesättigte oder ungesättigte, aliphatische und/oder heteroaliphatische Ether- oder Estergruppe mit 6 bis 40, vorzugsweise 10 bis 25 Kohlenstoffatomen; eine aromatische und/oder heteroaromatische Ether- oder Estergruppe mit 5 bis 40, vorzugsweise 10 bis 25 Kohlenstoffatomen oder eine Ether- oder Estergruppe mit 6 bis 40, vorzugsweise 10 bis 25 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist; vorzugsweise eine aliphatische und/oder aromatische Ether- oder Estergruppe mit 6 bis 40, vorzugsweise 10 bis 25 Kohlenstoffatomen, in der ein oder mehrere nicht benachbarte CH₂-Gruppen durch -R³C=CR³-, -C=C-,-(C=O)-, -(C=S)-, -(C=NR³),-, -C(=O)O-, -C(=O)NR³-, -NR³-, P(=O)(R³),-[CH(CₓH₂ₓ)P(=O)(OR¹)₂]-, -O-, -S-, -(SO)- oder (-SO₂)- ersetzt sein können, wobei R³ die zuvor für Formel (I) genannte Bedeutung aufweist und x eine ganze Zahl im Bereich von 0 bis 6, vorzugsweise 1 bis 5 ist.

Bevorzugt kann vorgesehen sein, dass ein Monomer gemäß Formel (III) insgesamt 1, 2 oder 3 Schwefelatome, besonders bevorzugt 1 oder 2 Schwefelatome und speziell bevorzugt genau ein Schwefelatom aufweist, wobei diese Zahl das In Formel (III) dargestellte Schwefelatom einschließt. Falls mehr als ein Schwefelatom enthalten ist, kann dieses in der Gruppe L¹, L³ und/oder V³ enthalten sein, vorzugsweise in Gruppe L¹ und/oder V³.

Bevorzugt kann vorgesehen sein, dass ein Monomer gemäß Formel (III) insgesamt 1, 2 oder 3 Phosphoratome, besonders bevorzugt 1 oder 2 Phosphoratome und speziell bevorzugt genau ein Phosphoratom aufweist, wobei diese Zahl das In Formel (III) dargestellte Phosphoratom einschließt. Falls mehr als ein Phosphoratom enthalten ist, kann dieses in der Gruppe L¹, L³ und/oder V³ enthalten sein, vorzugsweise in Gruppe L¹ und/oder V³.

Ferner kann vorgesehen sein, dass das Monomer mit mindestens einem Schwefelatom bevorzugt durch die Struktur (IIIa) darstellbar ist 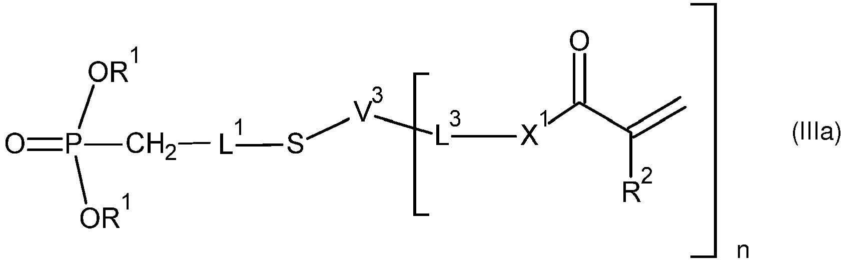 worin die Symbole n, L^{1a}, X¹, R¹ und R² die zuvor insbesondere für Formel (la) und die Gruppen L³ und V³ die zuvor insbesondere für Formel (III) genannte Bedeutung aufweisen

Ferner kann vorgesehen sein, dass die Gruppe L³ durch eine Struktur der Formel (L³-1) darstellbar ist 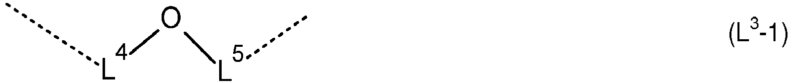 worin L⁴ und L⁵ bei jedem Auftreten unabhängig eine lineare, verzweigte oder cyclische Alkylengruppe mit 2 bis 20, vorzugsweise 3 bis 15 Kohlenstoffatomen ist, in der ein oder mehrere nicht benachbarte CH₂-Gruppen durch -R³C=CR³-, -C≡C-, C=O, C=S, C=NR³, -C(=O)O-, -C(=O)NR³-, NR³, P(=O)(R³), -[CH(CₓH₂ₓ)P(=O)(OR¹)₂]-, -O-, -S-, SO oder SO₂ ersetzt sein können, wobei R¹ und R³ die zuvor für Formel (I) genannte Bedeutung haben und x eine ganze Zahl im Bereich von 0 bis 6, vorzugsweise 1 bis 5 ist, oder eine Aryl- oder Heteroaryl-Gruppe mit 5 bis 20 Kohlenstoffatomen, die durch ein oder mehrere Substituenten R³ substituiert sein kann; oder eine Gruppe mit 6 bis 40, vorzugsweise 10 bis 25 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist; und die gestrichelten Linien jeweils die Bindung an die Gruppe V³ oder die Gruppe X¹ darstellen.

Vorzugsweise kann vorgesehen sein, dass die in Formel L³-1 dargelegte Gruppe L⁴ durch eine Struktur der Formel (L⁴-1) oder (L⁴-2) darstellbar ist 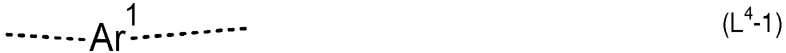 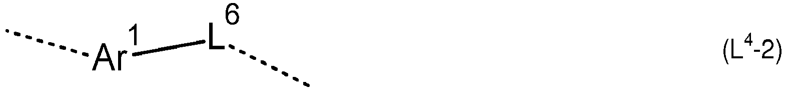 worin Ar¹ eine Aryl- oder Heteroaryl-Gruppe mit 4 bis 12, vorzugsweise 5 bis 10 Kohlenstoffatomen, vorzugsweise 6 Kohlenstoffatomen ist, die durch ein oder mehrere Substituenten R³ substituiert sein kann; L⁶ ausgewählt ist aus C=O, C=S, C=NR³, -C(=O)O-, -C(=O)NR³-, NR³, P(=O)(R³), -O-, -S-, SO oder SO₂ oder eine lineare, verzweigte oder cyclische Alkylengruppe mit 1 bis 6 Kohlenstoffatomen, in der ein oder mehrere nicht benachbarte CH₂-Gruppen durch -R³C=CR³-, -C≡C-, C=O, C=S, C=NR³, -C(=O)O-, -C(=O)NR³-, NR³, P(=O)(R³), -[CH(CₓH₂ₓ)P(=O)(OR¹)₂]-, -O-, -S-, SO oder SO₂ ersetzt sein können, wobei R¹ und R³ die zuvor für Formel (I) genannte Bedeutung haben, x eine ganze Zahl im Bereich von 0 bis 6, vorzugsweise 1 bis 5 ist, und die gestrichelten Linien jeweils die Bindung an die Gruppe V³ oder das Sauerstoffatom darstellen.

Vorzugsweise kann die Gruppe L⁵ durch eine Struktur der Formel (L⁵-1) darstellbar sein 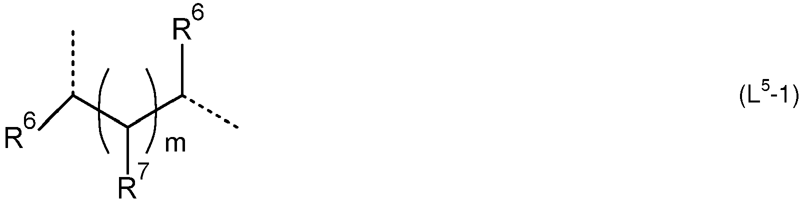 worin R⁶ bei jedem Auftreten unabhängig H, eine Alkylgruppe mit 1 bis 10 Kohlenstoffatomen oder eine aromatische Gruppe mit 6 bis 8 Kohlenstoffatomen, vorzugsweise H ist; R⁷ H oder eine Alkylgruppe mit 1 bis 10 Kohlenstoffatomen, vorzugsweise H ist; und der Index m eine ganze Zahl im Bereich von 0 bis 8, vorzugsweise 0 bis 7 und besonders bevorzugt 0 bis 5 ist und die gestrichelten Linien die Bindungen an das Sauerstoffatom oder die Gruppe X¹ darstellen.

Ferner kann vorgesehen sein, dass die Gruppe V³ durch eine Struktur der Formeln (V³-1), (V³-2), (V³-3), (V³-4), (V³-5) oder (V³-6) darstellbar ist 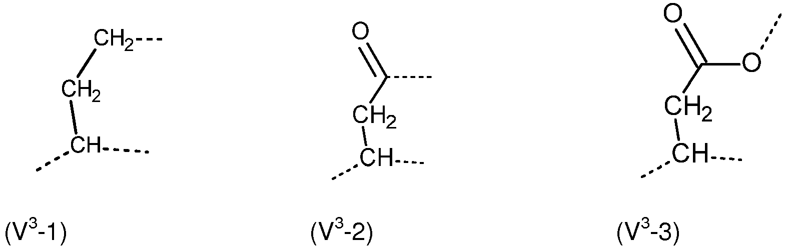 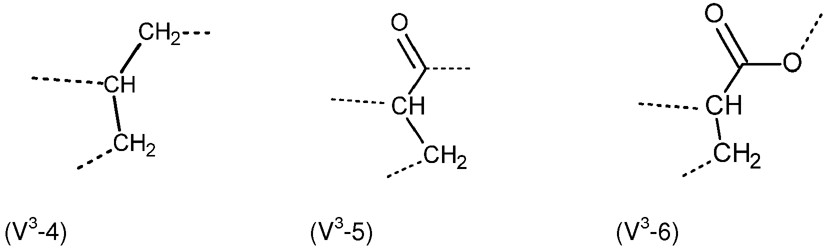 wobei die gestrichelten Linien die Bindungen an S oder die Gruppen L³ darstellen.

Bevorzugte Monomere mit mindestens einem Schwefelatom der vorliegenden Erfindung sind unter anderem durch die Formeln (IV), (V), (VI) und (VII) darstellbar 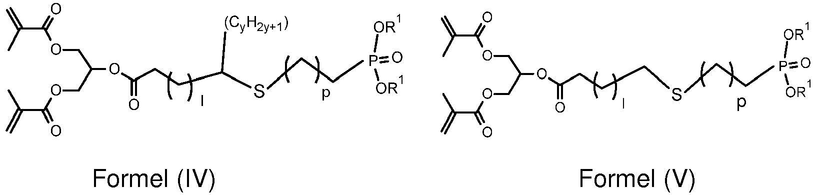 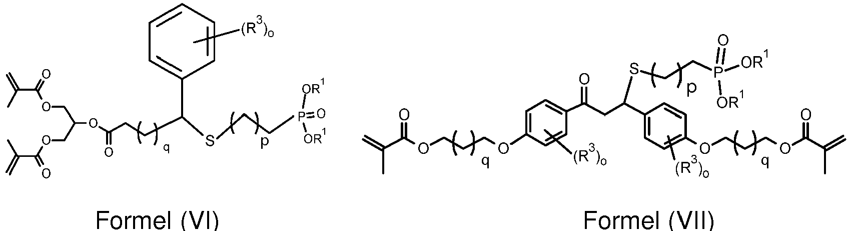 worin der Index I 0, 1, 2, 3, 4, 5, 6 oder 7, vorzugsweise 0, 1, 2, 3, 4, 5 oder 6 und besonders bevorzugt 0, 1, 2, 3, oder 4; der Index o 0, 1, 2, 3 oder 4, vorzugsweise 0, 1 oder 2, besonders bevorzugt 0 oder 1; der Index p 1, 2, 3, 4, 5, 6 oder 7, vorzugsweise 1, 2, 3, 4 oder 5 und besonders bevorzugt 2 oder 3; der Index q 1, 2, 3, 4, 5, 6 oder 7, vorzugsweise 1, 2, 3, 4 oder 5 und besonders bevorzugt 1 oder 2; und der Index y 0, 1, 2, 3, 4, 5, 6 oder 7, vorzugsweise 0, 1, 2, 3, 4, 5 oder 6 und besonders bevorzugt 0, 1, 2, 3, oder 4 ist und die Symbole R¹ und R³ die für Formel (I) genannte Bedeutung aufweisen.

Besonders bevorzugte Monomere sind durch die Formeln (IVa), (Va), (VIa) und (VIIa) darstellbar 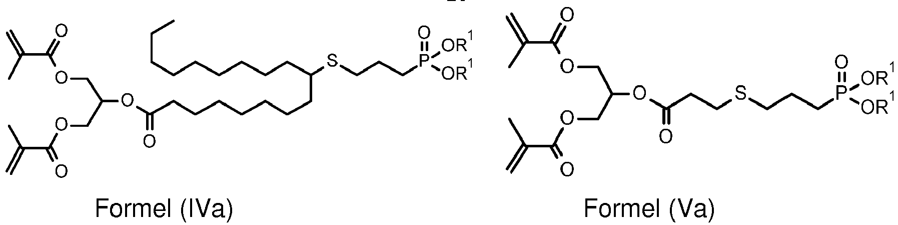 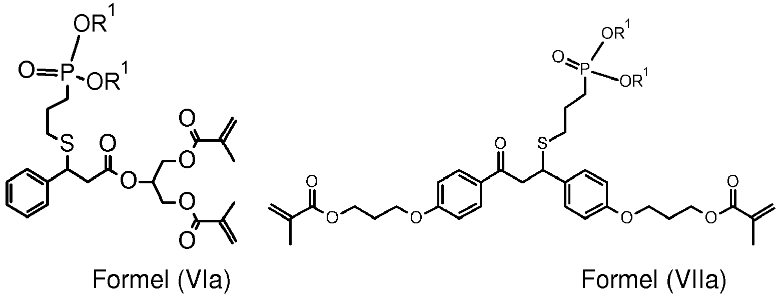 worin Symbole R¹ die für Formel (I) genannte Bedeutung aufweisen, wobei H besonders bevorzugt ist.

Die Monomere der Formel (I) lassen sich durch eine Kombination von an sich bekannten Verfahrensschritten aus bekannten Verbindungen herstellen. So kann ausgehend von einem Phosphonsäureesterderivat, welches mit einer Thiocarbonsäureverbindung zu einer Thioesterverbindung umgesetzt wird, ein Thiol mit einer Phosphonsäureestergruppe erhalten werden. Das Thiol mit einer Phosphonsäureestergruppe kann anschließend mit einer ungesättigten Verbindung in einer radikalischen Reaktion zu einem Thioether reagiert werden, der entweder direkt eine erfindungsgemäße Verbindung darstellt oder in einer Veresterungsreaktion zu einer erfindungsgemäßen Verbindung umgesetzt wird. Hierbei erfährt der Fachmann aus den Beispielen wertvolle Hinweise.

Ein bevorzugtes Verfahren zur Herstellung eines Monomers der Formel (I) mit mindestens einem Schwefelatom ist dadurch gekennzeichnet, dass man eine ungesättigte Verbindung mit einer schwefelhaltigen Verbindung gemäß Formel (E-I) umsetzt 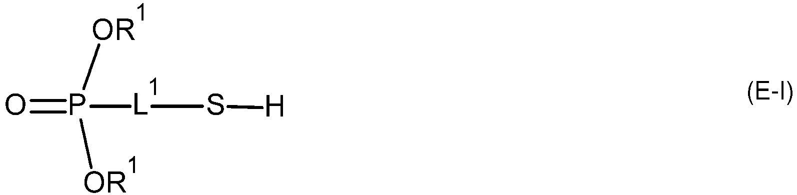 worin die Symbole L¹, und R¹ die zuvor, insbesondere für Formel (I) dargelegte Bedeutung aufweisen.

Ein weiterer Gegenstand der vorliegenden Erfindung ist eine härtbare Zusammensetzung zur Verwendung in der Dentalmedizin, umfassend mindestens ein erfindungsgemäßes Monomer.

Eine härtbare Zusammensetzung kann neben den zuvor näher dargelegten erfindungsgemäßen Monomeren mit mindestens einem Schwefelatom mindestens einen Initiator für eine radikalische Polymerisation der Monomere umfassen. Vorzugsweise kann eine härtbare Zusammensetzung Monomere umfassen, die nicht durch Formel (I) oder eine bevorzugte Ausgestaltung dieser Formel darstellbar sind. Ferner kann eine härtbare Zusammensetzung organische oder anorganische Füllstoffe enthalten.

Zu den bevorzugten radikalisch polymerisierbaren Monomeren, die nicht durch Formel (I) oder eine bevorzugte Ausgestaltung dieser Formel darstellbar sind, (Co-Monomere) gehören insbesondere mono- oder polyfunktionelle (Meth)acrylsäurederivate. Unter monofunktionellen (Meth)acrylsäurederivaten werden Verbindungen mit einer, unter polyfunktionellen (Meth)acrylsäurederivaten Verbindungen mit zwei oder mehr, vorzugsweise 2 bis 4 (Meth)acrylsäuregruppen verstanden. Polyfunktionelle Monomere haben eine vernetzende Wirkung.

Erfindungsgemäß bevorzugte mono- oder polyfunktionelle (Meth)acrylsäurederivate sind Methyl-, Ethyl-, Hydroxyethyl-, Butyl-, Benzyl-, Tetrahydrofurfuryl- oder Isobornyl (meth)acrylat, Bisphenol-A-di(meth)acrylat, Bis-GMA (ein Additionsprodukt aus Methacrylsäure und Bisphenol-A-diglycidylether), UDMA (ein Additionsprodukt aus 2-Hydroxyethylmethacrylat (HEMA) und 2,2,4-Trimethylhexamethylendiisocyanat (TMDI)), Di-, Tri- oder Tetraethylenglycoldi(meth)acrylat, Trimethylolpropantri(meth)acrylat, Pentaerythrittetra(meth)acrylat, Glycerindi(meth)acrylat, Glycerintri(meth)acrylat, 1,4-Butandioldi(meth)acrylat, 1,10-Decandioldi(meth)acrylat und 1,12-Dodecandioldi(meth)acrylat. Der Ausdruck (Meth)acrylat steht dabei für Methacrylat, Acrylat und Mischungen von Methacrylat und Acrylat.

Besonders bevorzugte mono- oder polyfunktionelle (Meth)acrylsäurederivate sind N-mono- oder -disubstitiuierte Acrylamide wie N-Ethylacrylamid, N,N-Dimethacrylamid, N-(2-Hydroxyethyl)acrylamid oder N-Methyl-N-(2-hydroxyethyl)acrylamid, N-monosubstituierte Methacrylamide wie N-Ethylmethacrylamid oder N-(2-Hydroxyethyl)methacrylamid sowie N-Vinylpyrrolidon und Allylether. Diese Monomere zeichnen sich durch eine hohe Hydrolysestabilität aus und eignen sich aufgrund ihrer relativ geringen Viskosität besonders als Verdünnermonomere.

Bevorzugte polyfunktionelle (Meth)acrylsäurederivate mit hoher Hydrolysestabilität sind vernetzende Pyrrolidone wie 1,6-Bis(3-vinyl-2-pyrrolidonyl)-hexan, Bisacrylamide wie Methylen- oder Ethylenbisacrylamid und Bis(meth)acrylamide wie N,N'-Diethyl-1, 3-bis(acrylamido)-propan,1, 3-Bis(methacrylamido)-propan, 1,4-Bis (acrylamido)-butan oder 1,4-Bis(acryloyl)-piperazin, die durch Umsetzung aus den entsprechenden Diaminen mit (Meth)acrylsäurechlorid synthetisiert werden können.

Vorzugsweise werden Mischungen der vorstehend genannten Monomere verwendet.

Die erfindungsgemäßen härtbare Zusammensetzung, die sich insbesondere als Dentalwerkstoffe eignen, können neben den polymerisierbaren Monomeren mit mindestens einem Schwefelatom der Formel (I) und ggf. den oben genannten CoMonomeren vorzugweise auch zusätzliche radikalisch polymerisierbare, säuregruppenhaltige Monomere (Haftmonomere) enthalten. Bevorzugte Säuregruppen sind Carbonsäuregruppen, Phosphonsäuregruppen, Phosphorsäuregruppen und Sulfonsäuregruppen.

Bevorzugte Monomere mit polymerisierbaren Carbonsäuren sind Maleinsäure, Acrylsäure, Methacrylsäure, 2-(Hydroxymethyl)acrylsäure, 4-(Meth)acryloyloxyethyl-trimellitsäureanhydrid, 10-Methacryloyloxydecylmalonsäure, N-(2-Hydroxy-3-methacryloyloxypropyl)-N-phenylglycin und 4-Vinylbenzoesäure. Bevorzugte Monomere mit polymerisierbaren Phosphonsäuregruppen sind Vinylphosphonsäure, 4-Vinylphenylphosphonsäure, 4-Vinylbenzylphosphonsäure, 2-Methacryloyloxyethylphosphonsäure, 2-Methacrylamidoethylphosphonsäure, 4-Methacrylamido-4-methyl-pentylphosphonsäure, 2-[4-(Dihydroxyphosphoryl)-2-oxa-butyl]-acrylsäure, 2-[4-(Dihydroxyphosphoryl)-2-oxa-butyl]-acrylsäureethyl- und -2,4,6-trimethylphenylester.

Bevorzugte Monomere mit polymerisierbaren Phosphorsäuregruppen sind 2-Methacryloyloxypropylmono- oder -dihydrogenphosphat, 2-Methacryloyloxyethylmono- oder -dihydrogenphosphat, 2-Methacryloyloxyethylphenyl-hydrogenphosphat, Dipentaerythritolpentamethacryloyloxyphosphat, 10-Methacryloyloxydecyl-di-hydrogenphosphat, Phosphorsäuremono(1-acryloyl-piperidin-4-yl)-ester, 6-(Methacryl-amido)hexyldihydrogenphosphat und 1,3-Bis-(N-acryloyl-N-propyl-amino)-propan-2-yl-dihydrogenphosphat.

Bevorzugte Monomere mit polymerisierbaren Sulfonsäuregruppen sind Vinylsulfonsäure, 4-Vinylphenylsulfonsäure und 3-(Methacrylamido)propylsulfon-säure.

Außerdem enthalten die erfindungsgemäßen härtbaren Zusammensetzungen, die sich vorzugsweise als Dentalwerkstoffe eignen, vorzugsweise auch einen Initiator für die radikalische Polymerisation.

Zur Initiierung der radikalischen Photopolymerisation werden vorzugsweise Benzophenon, Benzoin sowie deren Derivate oder α-Diketone oder deren Derivate wie 9,10-Phenanthrenchinon, 1-Phenyl-propan-1,2-dion, Diacetyl- oder 4,4'-Dichlorbenzil eingesetzt. Besonders bevorzugt werden Campherchinon und 2,2-Dimethoxy-2-phenyl-acetophenon und ganz besonders bevorzugt α-Diketone in Kombination mit Aminen wie 4-(Dimethylamino)-benzoesäureester, N,N-Dimethylaminoethylmethacrylat, N,N- Dimethyl-sym.-xylidin oder Triethanolamin als Reduktionsmittel verwendet. Besonders geeignet sind auch Norrish-Typ-I-Photoinitiatoren, insbesondere Acyl- oder Bisacylphosphinoxide, Monoacyltrialkyl- oder Diacyldialkylgermanium-Verbindungen wie Benzoyltrimethylgermanium, Dibenzoyldiethylgermanium oder Bis(4-methoxybenzoyl)diethylgermanium. Dabei lassen sich auch Mischungen der verschiedenen Photoinitiatoren wie beispielsweise Dibenzoyldiethylgermanium in Kombination mit Campherchinon und 4-Dimethylaminobenzoesäureethylester einsetzen.

Als Initiatoren für eine bei Raumtemperatur durchgeführte Polymerisation werden vorzugsweise Redox-Initiatorkombinationen wie beispielsweise Kombinationen von Benzoylperoxid mit N,N-Dimethyl-sym.-xylidin oder N,N-Dimethyl-p-toluidin verwendet. Darüber hinaus sind auch Redoxsysteme bestehend aus Peroxiden oder Hydroperoxiden und solchen Reduktionsmitteln wie z.B. Ascorbinsäure, Barbituraten, Thioharnstoffderivaten oder Sulfinsäuren besonders geeignet.

Weiterhin können die erfindungsgemäß eingesetzten Zusammensetzungen zur Verbesserung der mechanischen Eigenschaften oder zur Einstellung der Viskosität vorzugsweise auch organische oder anorganische Füllstoffpartikel enthalten. Bevorzugte anorganische partikuläre Füllstoffe sind amorphe kugelförmige Materialien auf Basis von Oxiden wie ZrO₂ und TiO₂ oder Mischoxiden aus SiO₂, ZrO₂ und/oder TiO₂ mit einer mittleren durchschnittlichen Partikelgröße von 0,005 bis 2 µm, vorzugsweise 0,1 bis 1 µm, nanopartikuläre oder mikrofeine Füllstoffe wie pyrogene Kieselsäure oder Fällungskieselsäure mit einer mittleren durchschnittlichen Partikelgröße von 5 bis 200 nm, vorzugsweise 10 bis 100 nm, Minifüllstoffe wie Quarz-, Glaskeramik- oder Glaspulver mit einer durchschnittlichen Teilchengröße von 0,01 bis 10 µm, vorzugsweise 0,1 bis 1 µm, sowie röntgenopake Füllstoffe wie Ytterbiumtrifluorid oder nanopartikuläres Tantal (V) -oxid bzw. Bariumsulfat mit einer mittleren durchschnittlichen Partikelgröße von 10 bis 1000 nm, vorzugsweise 100 bis 300 nm.

Die durchschnittlichen Partikelgrößen beziehen sich auf das Zahlenmittel und können durch mikroskopische Verfahren, beispielsweise Rasterelektronenmikroskopie bestimmt werden. Bei sphärischen Partikeln beziehen sich die Größen auf den Durchmesser, bei faserförmigen auf die maximale Ausdehnung des jeweiligen Partikels.

Außerdem können die erfindungsgemäß eingesetzten Zusammensetzungen weitere Additive enthalten, vor allem Lösungsmittel wie Wasser, Ethanol oder Aceton bzw. entsprechende Lösungsmittelgemische, sowie beispielsweise Stabilisatoren, Aromastoffe, Farbmittel, mikrobiozide Wirkstoffe, fluoridionenabgebende Additive, optische Aufheller, Weichmacher oder UV-Absorber.

Besonders bevorzugt sind härtbare Zusammensetzungen, die sich bevorzugt als Dentalwerkstoffe eignen, auf Basis eines polymerisierbaren Monomeren mit mindestens einem Schwefelatom der Formel I und insbesondere auf Basis eines polymerisierbaren Monomeren der Formel II oder III, welche die folgenden Bestandteile enthalten:
a) 0,1 bis 50 Gew.-%, insbesondere 1 bis 40 Gew.-%, bevorzugt 2 bis 30 Gew.-% und besonders bevorzugt 5 bis 20 Gew.-% vernetzbares Monomer mit mindestens einem Schwefelatom der Formel I,
b) 0,01 bis 10 Gew.-%, bevorzugt 0,1 bis 3 Gew.-% und besonders bevorzugt 0,2 bis 2 Gew.-% Initiator,
c) 0 bis 80 Gew.-%, bevorzugt 1 bis 60 Gew.-% und besonders bevorzugt 5 bis 50 Gew.-% Co-Monomer,
d) 0 bis 30 Gew.-%, bevorzugt 0,5 bis 15 Gew.-% und besonders bevorzugt 1 bis 5 Gew.-% Haftmonomer,
e) 0 bis 80 Gew.-% Füllstoff und
f) 0 bis 70 Gew.-%, bevorzugt 0 bis 60 Gew.-% und besonders bevorzugt 0 bis 50 Gew.-% Lösungsmittel.

Der bevorzugte Füllstoffgehalt richtet sich dabei nach der gewünschten Anwendung. Adhäsive enthalten vorzugsweise 0 bis 20 Gew.-% und Zemente und Komposite vorzugsweise 20 bis 80 Gew.-% Füllstoff.

Dies gilt ebenso für den Lösungsmittelgehalt. Adhäsive enthalten vorzugweise bevorzugt 0 bis 60 Gew.-% und besonders bevorzugt 1 bis 50 Gew.-% Lösungsmittel. Dentalmaterialien, die Wasser als Lösungsmittel enthalten, sind bevorzugt. Besonders bevorzugt sind Dentalmaterialien, die 0 bis 20 Gew.-% und insbesondere 1 bis 10 Gew.-% Wasser enthalten.

Ferner ist die Verwendung eines erfindungsgemäßen Monomers oder einer härtbaren Zusammensetzung gemäß der vorliegenden Erfindung in der Dentalmedizin Gegenstand der vorliegenden Erfindung.

Die Erfindung wird im Folgenden anhand von Ausführungsbeispielen näher erläutert, ohne dass hierdurch eine Beschränkung erfolgen soll.

### Synthesebeispiele

Die eingesetzten Materialien wurden, wie nachfolgend dargelegt, kommerziell erhalten und ohne weitere Aufreinigung eingesetzt:
Allylbromid (99%, Acros Organics), Triethylphosphit (98%, Aldrich Chemistry), Thioessigsäure (98%, Acros Organics), Triethylamin (99%, Acros Organics), 2,2'-Azobis(2-methylpropionitril) (AIBN, 98%, Sigma-Aldrich), Trans-Zimtsäure (98+%, Acros Organics), 2,2-Dimethoxy-2-phenylacetophenon (99%, Aldrich), Ölsäure (Fluka), 3-Brom-1-propanol (97%, Alfa Aesar), *N,N'*-Dicyclohexylcarbodiimid (DCC, >99%, Fluka), *N,N*-dimethylpyridin-4-amin (DMAP, >98%, Fluka), Bromtrimethylsilan (98%, Acros Organics), Glycerin-1,3-dimethacrylat (Isomerenmischung, technische Qualität, 85 %, Aldrich), 4-Hydroxyacetophenon (99%, Alfa Aesar), 4-Hydroxybenzaldehyd (99%, Acros Organics), *N,N*-Dimethylacrylamid (99%, Acros Organics), zweifach destilliertes Wasser (Carl Roth), Dichlormethan (Fisher Chemical), Tetrahydrofuran (THF, p.a., VWR Chemicals), Salzsäure (37%, VWR Chemicals), Toluol (p.a., Sigma Aldrich), Phenothiazin (98%, Lancaster), Methacryloylchlorid (97%, Alfa Aesar) und Acryloylchlorid (97%, Fluka).

Diethyl(3-mercaptopropyl)phosphonat wurde durch literaturbekannte Verfahren erhalten gemäß folgendem Schema erhalten: 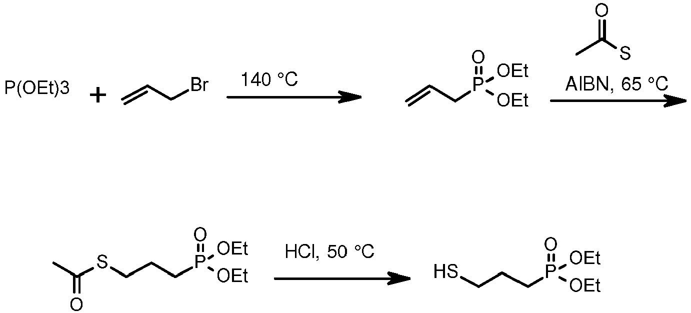 (Boutevin, B.;Hervaud, Y.;Mouledous, G.;Pelaprat, N. Phosphorus, Sulfur, and Silicon and the Related Elements 1998, 140, 125-133; Fourgeaud. Tetrahedron 2010, 66, 758; Putvinski, T.;Schilling, M.L.;Katz, H.E.;Chidsey, C.E.;Mujsce, A.;Emerson, A. Langmuir 1990, 6, 1567-1571)

Die erhaltenen Verbindungen wurden mit den nachfolgenden Verfahren charakterisiert:
¹H NMR Messungen wurden auf einem Bruker AVIII-300 Gerät bei 300.13 MHz durchgeführt. Die δ Skala bezieht sich auf Tetramethylsilan und wurde auf einen Wert von δ = 7.26 ppm für gelöstes CDCl₃ kalibriert. ¹³C NMR Messungen wurden bei 75 MHz, ³¹P NMR Messungen at 121 MHz durchgeführt.

Infrarot (IR) Spektren wurden bei Raumtemperatur mit einem Nicolet 6700 FT-IR Spektrometer aufgenommen, welches mit einer ATR-Einheit ausgestattet war. Die Messungen wurden im Bereich von 4000-300 cm⁻¹ durchgeführt.

Ein mit einem auf Elektronenstoßionisation basierenden Massenspektrometer gekoppelten Gaschromatograph (GC/MS (EI)) wurde zur Analyse eingesetzt, wobei die Messung auf einem GC/MS-System durchgeführt wurde ("Triple quadrupole ion trap mass spectrometer" von Finnigan Trace DSQ und ein Finnigan Trace GC Ultra). Das Gerät wurde auf einen m/z Bereich von 4000 Da kalibriert.

Die Reinheit der Verbindungen wurde durch ein Massenspektrometer unter Verwendung eines Ionenfallen-Massenspektrometer (Finnigan LCQ Deca (Thermo Quest)) überprüft. Die Ionisierung wurde mit einem Elektronenspray durchgeführt.

### Beispiel 1

### Schritt 1

### Synthese von 9-((3-Diethoxyphosphoryl)thio)octadecansäure

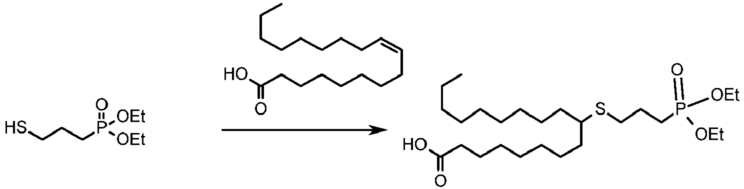

6.5 g (23 mmol) Ölsäure, 5.1 g (24 mmol) Diethyl(3-mercaptopropyl)phosphonat und 295.8 mg (1,15 mmol) 2,2-Dimethoxy-2-phenylacetophenon wurden gemischt. Die Mischung wurde 6,5 Stunden unter UV-Licht gerührt. Edukte, die nicht reagiert haben, wurden durch eine 1-molare Salzsäurelösung extrahiert (3x). Das erhaltene Rohprodukt wurde über eine Säulenchromatographie (Ethylacetat/Methanol 95/5) gereinigt. Eine gelbe Flüssigkeit wurde erhalten.

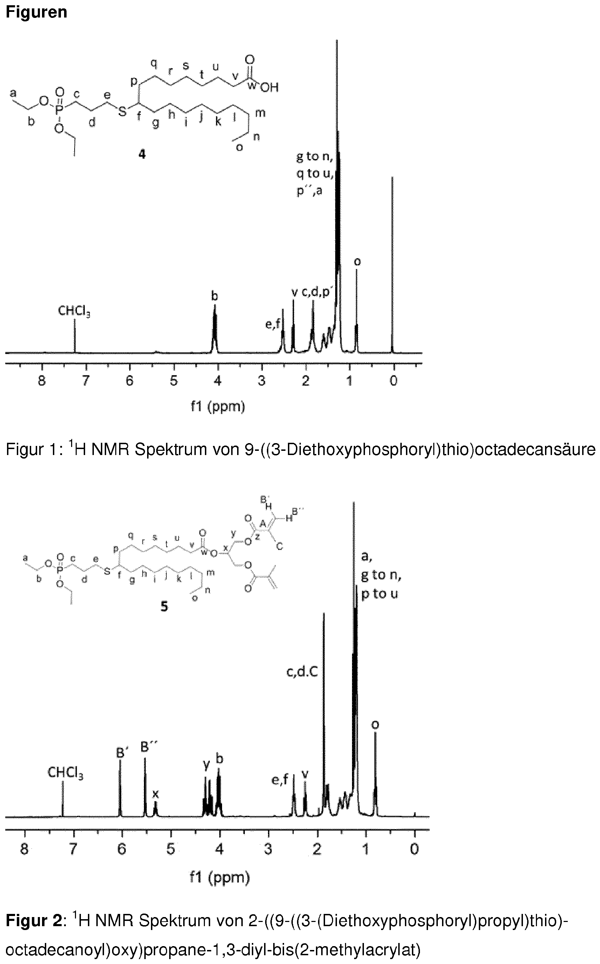

Das erhaltene ¹H NMR Spektrum von 9-((3-Diethoxyphosphoryl)thio)octadecansäure ist in Figur 1 abgebildet.
Ausbeute: 72 %;
R_{f}-Wert: 0.35 (Ethylacetat/Methanol 95/5);
FT-IR (diamond, cm⁻¹): ṽ = 3457 (b, v_{OH}), 2924/ 2853 (m, v_{C-H}), 1724 (w, v_{COOR}), 1456 (w, δ_{P-C}), 1393, 1205 (m, v_{P-O}), 1097, 1055 (m, δ_{P-O-C}), 1024 (s, δ_{P-O-C}), 962, 784 (m, v_{P-O-C}), 723, 540, 494;
¹H-NMR (300 MHz, CDCl₃, δ [ppm]): 4,12 - 3 ,97 (m, 4H, b), 2,58 - 2,42 (m, 3H, e and f), 2,25 (t, ³J_{HH}= 7,39 Hz, 2H, v), 1,91 - 1,71 (m, 5H, c, d and p'), 1,61- 1,16 (m, 34H, g bis n, q bis u, p" and a), 0,85 - 0,79 (m, 3H, o);
¹³C-NMR (75 MHz, CDCl₃, δ [ppm]): 176,68 (s, w), 60,79 (d, ²J_{PC} = 6,56 Hz, b), 44,86 (s, f), 33,96 - 33,71 (d, p and g), 33,11 (s, v), 30,88 (s, m), 29,86 (d, ³J_{PC} = 18,19 Hz, e), 28,65 - 27,92 (m, i bis l and r bis t), 25,83 - 25,57 (m, h and q), 23,79 (s, u), 23,67 (d, ¹J_{PC} = 141,57 Hz, c), 21,82 (d, ²J_{PC} = 4,57 Hz, d), 21,66 (s, n), 15,42 (s,
³J_{PC} = 6,08 Hz, a), 13,09 (s, o);
³¹P-NMR (121 MHz, CDCl₃, δ [ppm]): 32,09 and 32,03 (2s, Markownikow and Anti-Markownikow product);
MS (ESI) m/z: 495 [M + H⁺]; EA_{calc}. (%): C 60,70, H 10,39, S 6,48; EA_{found} (%): C 60,37, H: 10,14, S: 6,11.

### Schritt 2:

### Synthese von 2-((9-((3-(Diethoxyphosphoryl)propyl)thio)octadecanoyl)oxy)propane-1,3-diyl-bis (2-methylacrylat)

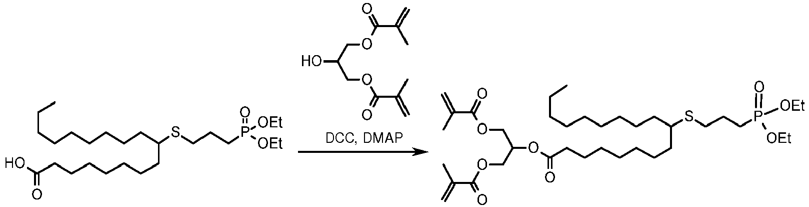

In einem 50 ml Kolben wurden 2 g (4 mmol) 9-((3-Diethoxyphosphoryl)thio) octadecansäure in 20 ml trockenem Dichlormethan gelöst und in einem Eisbad auf 0°C gekühlt. 0,83 (4 mmol) DCC und 50 mg (0,4 mmol) DMAP wurden in 5 ml trockenem Dichlormethan suspendiert. Die Suspension wurde tropfenweise zugegeben. Nach 2 Stunden wurden 0.9 ml (1 g, 4,4 mmol) Glycerin-1,3-dimethacrylat zugegeben; die Mischung wurde auf Raumtemperatur gebracht und 24 Stunden gerührt. Ausgefallenes DCU wurde filtriert und das Filtrat wurde mit einem Trockeneis-Aceton-Gemisch 45 Minuten gekühlt (3x). Die organische Lösung wurde unter vermindertem Druck konzentriert. Das Rohprodukt wurde durch Säulenchromatographie (Silikagel) mit Ethylacetat gereinigt. Eine gelbe Flüssigkeit mit honigartiger Konsistenz wurde erhalten.

Das erhaltene ¹H NMR Spektrum von 2-((9-((3-(Diethoxyphosphoryl)propyl)thio)-octadecanoyl)oxy)propane-1,3-diyl-bis(2-methylacrylat) ist in Figur 2 abgebildet.
Ausbeute: 39 %;
R_{f}-Wert: 0.66 (Ethylacetat);
FT-IR (diamond, cm⁻¹): ṽ = 2926/ 2854 (w, v_{C-H}), 1723 (s, v_{COOR}), 1638, 1454 (w, δ_{P-C}), 1321, 1294, 1235 (m, v_{P-O}), 1154 (s, δ_{P-O-C}), 1097, 1057, 1027 (s, δ_{P-O-C}), 942, 812 (m, v_{P-O-C}), 723, 653, 541, 491;
¹H-NMR (300 MHz, CDCl₃, δ [ppm]) = 6,06 - 6,03 (m, 2H, B'), 5,55 - 5,51 (m, 2H, B"), 5,38 - 5,26 (m, 1H, x), 4,35 - 4,14 (m, 4H, y), 4,10 - 3,95 (m, 4H, b), 2,53 - 2,44 (m, 3H, e und f), 2,26 (td, ²J_{HH} = 1,99 Hz, ³J_{HH}= 7,51 Hz, 2H, v), 1,88 - 1,75 (m, 10H, c, d und C), 1,60 - 1,20 (m, 34H, a, g bis n und p bis u), 0,85 - 0,77 (m, 3H, o);
¹³C-NMR (75 MHz, CDCl₃, δ [ppm]) = 172,72 (s, w), 166,66 (s, z), 135,61 (s, A), 126,25 (s, B), 68,73 (s, x), 62,55 (s, y), 60,51 (d, ²J_{PC} = 6,64 Hz, b), 45,,79 (s, f), 34,79 (s, p und g), 34,04 (s, v), 31,82 (s, m), 30,82 (d, ³J_{PC} = 17,94 Hz, e), 29,59 - 28,97 (m, i bis l und r bis t), 26,72 (s, h und q), 24,83 (s, u), 24,70 (d, ¹J_{PC} = 141,55 Hz, c), 22,84 (d, ²J_{PC} = 4,61 Hz, d), 22,60 (s, n), 18,17 (s, C), 16,40 (s, ³J_{PC} = 6,05 Hz, a), 14,05 (s, o);
³¹P-NMR (121 MHz, CDCl₃, δ [ppm]) = 31,68 (s, 1P);
MS (ESI) m/z: 495 [C₂₅H₅₁O₅PS + H⁺], 706 [M + H⁺]; EA_{calc}. (%): C 61, 34, H 9,29, S 4,55; EA_{found} (%): C 61,17, H 9,26, S 4,12.

### Beispiel 2

### Synthese von 3-((1-((1,3-Bis(methacryloyloxy)propan-2-yl)oxy)-1-oxooctadecan-9-yl(thio)propyl)phosphonsäure

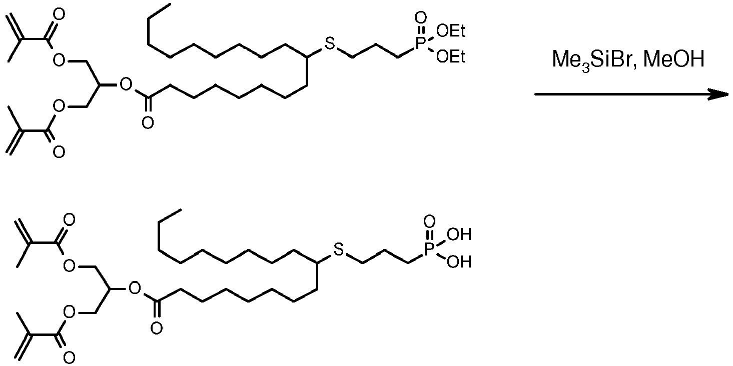

Die Hydrolyse von 2-((9-((3-(Diethoxyphosphoryl)propyl)thio)octadecanoyl)oxy)-propane-1,3-diyl-bis(2-methylacrylat) wurde mittels bekannter Verfahren durchgeführt.

570 mg (0,81 mmol) 2-((9-((3-(Diethoxyphosphoryl)propyl)thio)octadecanoyl)oxy)-propane-1,3-diyl bis 2-methylacrylat wurde in 8,2 ml trockenem Dichlormethan gelöst. Die Lösung wurde durch Einleiten von Stickstoff über 10 Minuten entgast. Anschließend wurden 1,07 µl (8,1 mmol) Bromtrimethylsilan zugegeben und die Lösung wurde bei einer Badtemperatur von 23°C für 9 Stunden gerührt. Das organische Lösungsmittel wurde bei vermindertem Druck entfernt und unter Vakuum getrocknet. Das Zwischenprodukt wurde mit 15 ml Methanol behandelt und 1 Stunde bei Raumtemperatur gerührt. Anschließend wurde das Lösungsmittel bei vermindertem Druck entfernt. Nach Trocknung des Produkts unter Vakuum wurde eine gelbe hochviskose Flüssigkeit erhalten.

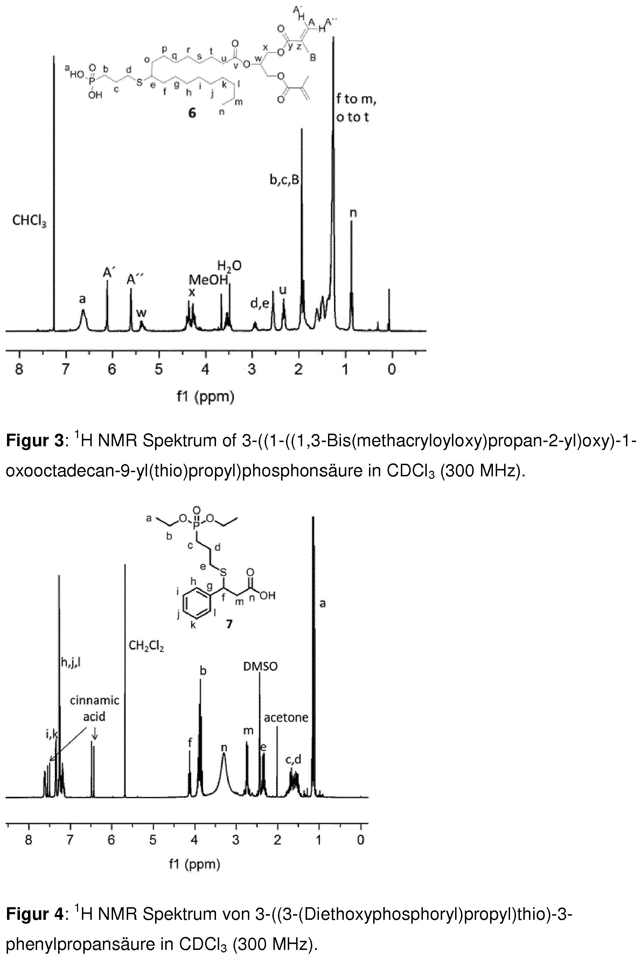

Das erhaltene ¹H NMR Spektrum von 3-((1-((1,3-Bis(methacryloyloxy)propan-2-yl)oxy)-1-oxooctadecan-9-yl(thio)propyl)phosphonsäure ist in Figur 3 abgebildet.
Ausbeute: 79 % (0.64 mmol);
FT-IR (diamond, cm⁻¹): ṽ = 3386 (b, v_{O-H}), 2925/ 2854 (m, v_{C-H}), 1724 (s, v_{COOH}), 1638 (w, v_{CO=C}), 1455 (w, δ_{P-C}), 1378, 1294 (m, v_{P-O}), 1154 (s, δ_{P-O-C}), 1007 (s, δ_{P-O-C}), 942, 813 (m, v_{P-O-C}), 723, 654, 520, 461;
¹H-NMR (300 MHz, CDCl₃, δ [pm]) = 6,56 (s, a), 6,07 - 6,04 (m, 2H, A'), 5,56 - 5,52 (m, 2H, A"), 5,36 - 5,22 (m, 1H, w), 4,41 - 4,11 (m, 4H, x), 2,53 - 2,44 (m, 3H, d und e), 2,26 (t, ²J_{HH} = 7,22 Hz, 2H, u), 1,90 - 1,79 (m, 10H, b, c und B), 1,54 - 1,14 (m, 28H, f bis m und o bis t), 0,83 - 0,79 (m, 3H, n);
¹³C-NMR (75 MHz, CDCl₃, δ [ppm]) = 172,97 (s, v), 166,79 (s, y), 135,64 (s, z), 126,40 (s, A), 68,82 (s, w), 62,62 (s, x), 45,91 (s, e), 34,76 (s, o und f), 34,19 (s, u), 31,88 (s, l), 30,81 (d, ³J_{PC} = 19,21 Hz, d), 29,68 - 29,03 (m, h bis k und q bis s), 26,74 (s, g und p), 24,90 (s, t), 24,89 (d, ¹J_{PC} = 143,37 Hz, b), 22,67 (s, m), 22,53 (d, ²J_{PC} = 4,17 Hz, c), 18,24 (s, B), 14,12 (s, n);
³¹P-NMR (121 MHz, CDCl₃, δ [ppm]) = 36,68 (s, 1P);
MS (ESI) m/z: 650 [M + H⁺], 678 [einfach verseiftes Edukt + H⁺], 729 [Edukt + Na⁺].

### Beispiel 3

### Schritt 1:

### Synthese von 3-((3-(Diethoxyphosphoryl)propyl)thio)-3-phenylpropansäure

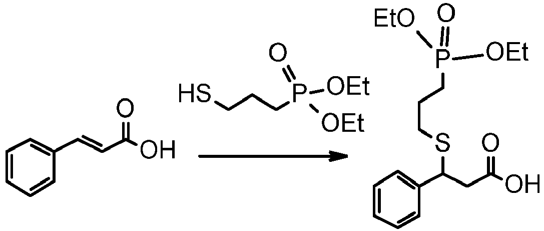

2 g (13,5 mmol) Zimtsäure und 2.56 g (12.1 mmol) Diethyl (3-mercaptopropyl)phosphonat wurden in 6 ml THF gelöst. Die Lösung wurde durch Einleiten von Stickstoff über 15 Minuten entgast. Nach Zugabe von 0,4 ml (2,64 mmol) DBU (1,8-Diazabicyclo[5.4.0]-7-undecen) wurde die Mischung ca. 48 Stunden unter Rückfluss erhitzt. Nach einer Abkühlung der Reaktionsmischung auf Raumtemperatur wurde Dichlormethan zugegeben und die Mischung wurde zweimal mit 1molarer, einmal mit 0,1molarer Salzsäure und mit Wasser gewaschen. Das Lösungsmittel wurde bei reduziertem Druck entfernt.

Das erhaltene ¹H NMR Spektrum von 3-((3-(Diethoxyphosphoryl)propyl)thio)-3-phenylpropansäure ist in Figur 4 abgebildet.
Rohausbeute: 2.09 g;
FT-IR (diamond, cm⁻¹): ṽ = 3465 (b, v_{OH}), 2981/ 2933/ 2905/ 2867 (w, v_{C-H}), 1707 (m, v_{COOH}), 1638 (w, v_{C=C}), 1575, 1494, 1451, 1393, 1366, 1306, 1261, 1190 (m, v_{C-OH}), 1097, 1020 (s, δ_{P-O-C}), 958 (s), 834, 796, 770/ 700 (m, δ_{benzyl}), 748, 699, 581, 530, 485;
¹H-NMR (300 MHz, DMSO-d6, δ [ppm]): 7,42 - 7,39 (m, 2H, i und k), 7,35 - 7,31 (m, 3H, h, j und l), 4,19 (t, ³J_{HH}= 7,67 Hz, 1H, f), 4,02 - 3,87 (m, 4H, b), 3,36 (s, n), 2,81 (dd, ³J_{HH} = 7,75 Hz, 2H, m), 2,47 - 2,33 (m, 2H, e), 1,89 - 1,50 (m, 4H, c und d), 1,20 (t, ³J_{HH}= 7,07 Hz, 6H, a);
³¹P-NMR (121 MHz, DMSO-d6, δ [ppm]): 31,29 (s, 1P Edukt]), 31,10 (s, 1P, Produkt);
MS (ESI) m/z: 361 [C₁₆H₂₅O₅PS + H⁺].

### Schritt 2:

### Synthese von 2-((3-((3-(Diethoxyphosphoryl)propyl)thio-3-phenylpropanoyl)oxy)-propan-1,3-diyl bis (2-methylacrylat)

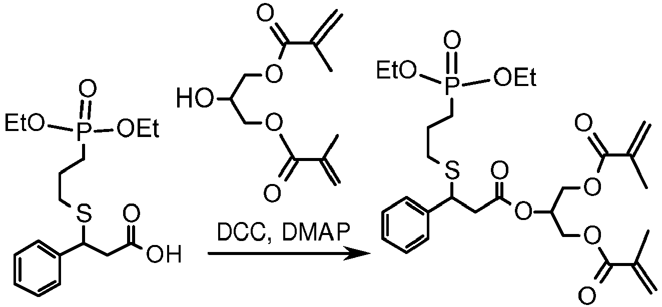

In einem 100 ml Kolben wurden 1 g (approx. 2.3 mmol) 3-((3-(Diethoxy-phosphoryl)propyl)thio)-3-phenylpropansäure in 11 ml trockenem Dichlormethan gelöst und in einem Eisbad auf 0°C gekühlt. 711,8 mg (3,5 mmol) DCC und 42 mg (0,35 mmol) DMAP wurden in 2 ml trockenem Dichlormethan suspendiert. Die Suspension wurde tropfenweise mit einer Spritze zugegeben. Nach 1,5 Stunden wurden 774 µl (3,8 mmol) Glycerin-1,3-dimethacrylat zugegeben; die Mischung wurde auf Raumtemperatur gebracht und 48 Stunden gerührt. Ausgefallenes DCU wurde filtriert und das Filtrat wurde mit einem Trockeneis-Aceton-Gemisch 60 Minuten gekühlt (3x). Die organische Lösung wurde unter vermindertem Druck konzentriert. Das Rohprodukt wurde durch Säulenchromatographie (Silikagel) mit Ethylacetat und leichtem Petroleum (v:v 1:1) gereinigt. Eine leicht gelbe Flüssigkeit mit honigartiger Konsistenz wurde erhalten.

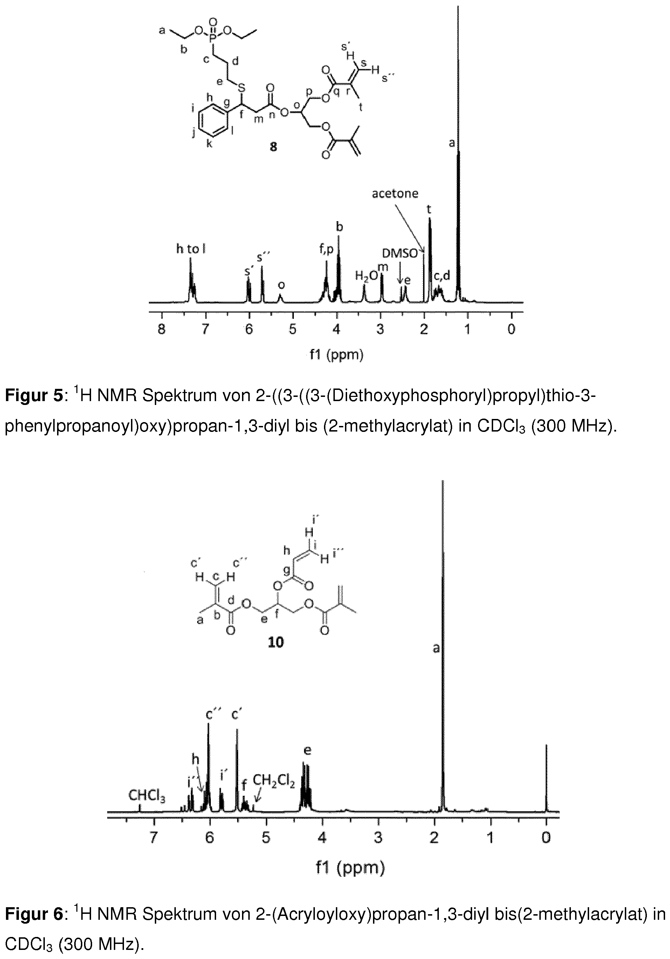

Das erhaltene ¹H NMR Spektrum von 2-((3-((3-(Diethoxyphosphoryl)propyl)thio-3-phenylpropanoyl)oxy)propan-1,3-diyl bis (2-methylacrylat) ist in Figur 5 abgebildet.
Ausbeute: 53 % (1.2 mmol);
R_{f}-Wert: 0,3 (Ethylacetat: leichtes Petroleum 1:1);
FT-IR (diamond, cm⁻¹): ṽ = 2961/ 2957/ 2928/ 2902 (w, v_{C-H}), 1720 (m, v_{C=O}), 1636 (w, v_{C=C}), 1453, 1377, 1321, 1294, 1234, (m, v_{P=O}), 1144, 1096, 1055, 1025 (s, δ_{P-O-}c), 950, 812/ 700 (m, δ_{monosubstituted ring}), 653, 532 cm⁻¹;
¹H-NMR (300 MHz, DMSO-d6, δ [ppm]): 7,36 - 7,18 (m, 5H, h bis l), 6,03 - 5,95 (m, 2H, s'), 5,71 - 5,65 (m, 2H, s"), 5,32 - 5,20 (m, 1H, o), 4,37 - 4,14 (m, 5H, f und p), 4,06 - 3,87 (m, 4H, b), 2,95 (dd, ³J_{HH}= 7,45 Hz, 2H, m), 2,46 - 2,32 (m, 2H, e), 1,88 - 1,82 (m, 6H, t), 1,79 - 1,52 (m, 4H, c und d), 1,20 (t, ³J_{HH}= 7,08 Hz, 6H, a); ¹³C-NMR (75 MHz, DMSO-d6, δ [ppm]): 169,30 (s, n), 165,97 (s, q), 141,11 (s, g), 135,25 (s, r), 128,33 - 126,32 (quintet, h bis l), 127,17 (s, s), 69,05 (s, o), 62,19 (d, ²J_{PC} = 28,03 Hz, b), 60,79 (s, p), 44,25 (s, m), 40,36 (s, f), 30,74 (d, ³J_{PC} = 17,55 Hz, e), 23,52 (d, ¹J_{PC} = 138,96 Hz, c), 22,15 (d, ²J_{PC} = 4,16 Hz, d), 17,18 (s, t), 16,19 (d, ³J_{PC} = 5,8 Hz, a);
³¹P-NMR (121 MHz, DMSO-d6, δ [ppm]): 30,97 (s, 1P);
MS (ESI) m/z: 572 [C₂₇H₃₉O₉PS + H⁺]; EA_{calc.} (%): C 56,83, H 6,89, S 5,62; EA_{found} (%): C 56,43, H 6,95, S 5,54.

### Beispiel 4

### Synthese von (3-((3-1,3-Bis(methacryloyloxy)propan-2-yl)oxy-1-phenylpropyl)thio)propyl)-phosphonsäure

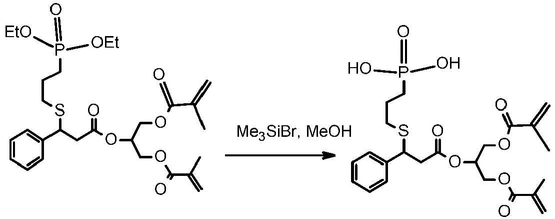

Gemäß dem in Beispiel 2 beschriebenen Verfahren wurde aus 2-((3-((3-(Diethoxyphosphoryl)propyl)thio-3-phenylpropanoyl)oxy)propan-1,3-diyl-bis-(2-methylacrylat) 3-((3-1,3-Bis(methacryloyloxy)propan-2-yl)oxy-1-phenylpropyl)thio)-propyl)phosphonsäure hergestellt.

### Beispiel 5

### Schritt 1:

### Synthese von 2-(Acryloyloxy)propan-1,3-diyl bis(2-methylacrylat)

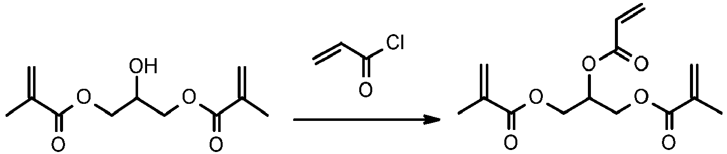

2-(Acryloyloxy)propan-1,3-diyl bis(2-methylacrylat) wurde durch Zugabe von 9,2 ml (10,3 g, 45 mmol) Glycerin-1,3-dimethacrylat und 7,3 ml (5,3 g, 53 mmol) Triethylamin zu 100 ml trockenem Dichlormethan in einem 250-ml Kolben erhalten. Die Lösung wurde in einem Eisbad auf 0°C gekühlt. Anschließend wurden 4,3 ml (4,8 g, 53 mmol) Acrylsäurechlorid, gelöst in 30 ml trockenem Dichlormethan, tropfenweise über 30 Minuten zugegeben. Die Lösung wurde auf Raumtemperatur gebracht und über 72 Stunden gerührt. Die organische Lösung wurde dreimal mit Wasser gewaschen, über MgSO₄ getrocknet und filtriert. Nach Zugabe von Phenothiazin wurde das Lösungsmittel bei vermindertem Druck entfernt und unter Vakuum getrocknet. Eine braune Flüssigkeit wurde erhalten.

Das erhaltene ¹H NMR Spektrum von 2-(Acryloyloxy)propan-1,3-diyl bis(2-methylacrylat) ist in Figur 6 abgebildet.
Ausbeute: 83 % (37,6 mmol);
FT-IR (diamond, cm⁻¹): ṽ = 2980/ 2960/ 2929 (w, v_{C-H}), 1719 (s, v_{COOR}), 1680, 1637 (w, v_{C=C}), 1453, 1406, 1321, 1293, 1264, 1149, 1050, 1066, 1043, 1012, 984 (m, δ_{C=C}), 943, 862, 809, 657, 598, 460;
¹H-NMR (300 MHz, CDCl₃, δ [ppm]): 6,35 (ddd, ²J_{HH} = 5,10 Hz, ³J_{HH} = 17,08 Hz, 1H, i"), 6,16 - 5,98 (m, 3H, h und c"), 5,80 (ddd, ²J_{HH} = 3,92 Hz, ³J_{HH} = 10,4 Hz, 1H, i'), 5,52 (s, 1H, c'), 5,44 - 5,31 (m, 1H, f), 4,40 - 4,19 (m, 4H, e), 1,85 (s, 6H, a);
¹³C-NMR (75 MHz, CDCl₃, δ [ppm]): 165,68 (s, d), 164,03 (s, g), 134,66 (s, b), 130,77 (s, i), 126,80 (s, h), 125,29 (s, c), 68,16 (s, f), 61,53 (s, e), 17,18 (s, a);
MS (ESI) m/z: 99 [C₅H₇O₂˙], 197 [C₁₀H₁₃O₄˙], 211 [C₁₁H₁₅O₄˙], 214 [C₁₀H₁₄O₅], 283 [M + H⁺], 300 [M + NH₄⁺], 305 [M + Na⁺].

### Schritt 2:

### Synthese von 2-((3-((3-(Diethoxyphosphoryl)propyl)thio)propanoyl)oxy)propan-1,3-diyl bis(2-methylacrylat)

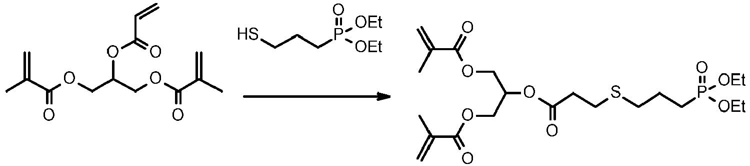

In einem Rundkolben wurden 5,1 g (18 mmol) 2-(Acryloyloxy)propan-1,3-diyl bis(2-methylacrylat), 3,1 g (14 mmol) Diethyl (3-mercaptopropyl)phosphonat und 123 µl Triethylamin bei Raumtemperatur 6 Tage gerührt. Anschließend wurden 132 µl konzentrierte Salzsäure zugegeben. Die Suspension wurde in Dichlomethan gelöst und dreimal mit Wasser gewaschen. Die organische Phase wurde über MgSO₄ getrocknet, filtriert und nach Zugabe von Phenothiazin unter vermindertem Druck konzentriert. Das Rohprodukt wurde durch Säulenchromatographie (Silikagel) mit Ethylacetat gereinigt. Eine gelborange Flüssigkeit mit honigartiger Konsistenz wurde erhalten.

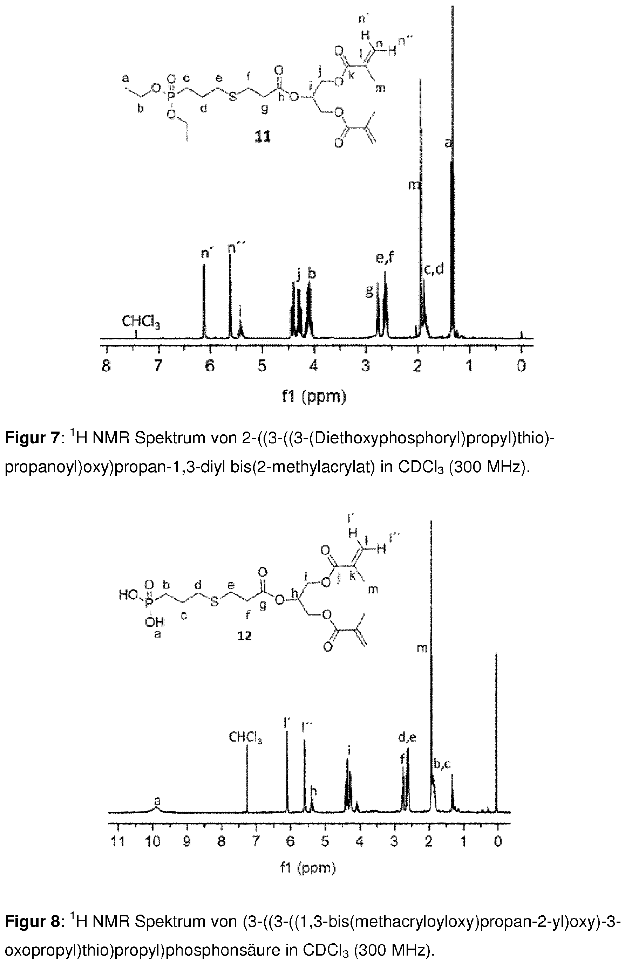

Das erhaltene ¹H NMR Spektrum von 2-((3-((3-(Diethoxyphosphoryl)propyl)thio)-propanoyl)oxy)propan-1,3-diyl bis(2-methylacrylat) ist in Figur 7 abgebildet.
Ausbeute: 35 % (5,1 mmol);
R_{f}-Wert: 0,39 (Ethylacetat);
FT-IR (diamond, cm⁻¹): ṽ = 2962/ 2929/ 2907 (w, v_{C-H}), 1722 (s, v_{COOR}), 1637 (w, v_{C=C}), 1452 (w, δ_{P-C}), 1407, 1321, 1295, 1258, 1235 (w, v_{P-O}), 1156 (m, δ_{P-O-C}), 1022 (m, δ_{P-O-C}), 962, 863, 792 (m, v_{P-O-C}), 701, 661, 539, 480;
¹H-NMR (300 MHz, CDCl₃, δ [ppm]): 6,13 - 6,11 (m, 2H, n'), 5,63 - 5,60 (m, 2H, n"), 5,45 - 5,35 (m, 1H, i), 4,44 - 4,25 (m, 4H, j), 4,16 - 4,04 (m, 4H, b), 2,77 (t, ³J_{HH} = 6,99 Hz, 2H, g), 2,63 (dd, ³J_{HH} = 6,99 Hz, 4H, e und f), 1,95 - 1,79 (m, 10H, c, d und m), 1,33 (t, ³J_{HH} = 7,13 Hz, 6H, a);
¹³C-NMR (75 MHz, CDCl₃, δ [ppm]): 169,85 (s, h), 165,68 (s, k), 134,63 (s, l), 125,4 (s, n), 68,42 (s, i), 61,46 (s, j), 60,55 (d, ²J_{PC} = 6,40 Hz, b), 33,68 (s, g), 31,52 (d, ³J_{PC} = 18,56 Hz, e), 25,59 (s, f), 23,53 (d, ¹J_{PC} = 141,68 Hz, c), 21,47 (d, ²J_{PC} = 4,57 Hz, d), 17,24 (s, m), 15,47 (d, ³J_{PC} = 6 Hz, a);
³¹P-NMR (121 MHz, CDCl₃, δ [ppm]): 31,23 (s, 1P);
MS (ESI) m/z: 495 [M + H⁺], 517 [M + Na⁺], 533 [C₂₁H₃₅O₉PS + K⁺]; EA_{calc.} (%): C 51,00, H 7, 13, S 6,48; EA_{found} (%): C 50,43, H 7,22, S 6,65.

### Beispiel 5

### Synthese von (3-((3-((1,3-bis(methacryloyloxy)propan-2-yl)oxy)-3-oxopropyl)thio)-propyl)phosphonsäure

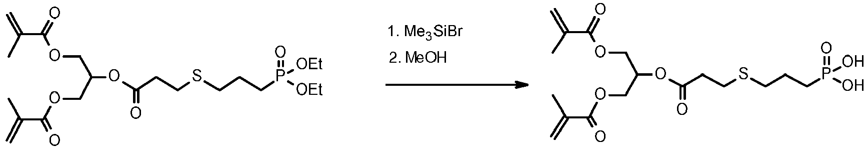

In einem 100 ml Zweihalsrundkolben wurden 2 g (4 mmol) 2-((3-((3-(Diethoxy-phosphoryl)propyl)thio)propanoyl)oxy)propan-1,3-diyl bis(2-methylacrylat) in 5,3 ml trockenem Dichlormethan gelöst. Die Lösung wurde durch Einleiten von Stickstoff über 1 Stunde entgast.

Anschließend wurden 5,3 ml (40 mmol) Bromtrimethylsilan zugegeben und die Lösung wurde bei einer Badtemperatur von 23°C für 24 Stunden gerührt. Das organische Lösungsmittel wurde bei vermindertem Druck entfernt und unter Vakuum getrocknet. Das Zwischenprodukt wurde mit 5 ml Methanol behandelt und 1 Stunde bei Raumtemperatur gerührt. Anschließend wurde das Lösungsmittel bei vermindertem Druck entfernt. Nach Trocknung des Produkts unter Vakuum wurde eine olivgrüne, klare, hochviskose Flüssigkeit erhalten.

Das erhaltene ¹H NMR Spektrum von (3-((3-((1,3-bis(methacryloyloxy)propan-2-yl)oxy)-3-oxopropyl)thio)propyl)phosphonsäure ist in Figur 8 abgebildet.
Ausbeute: 90 % (3,6 mmol);
FT-IR (diamond, cm⁻¹): ṽ = 3498 (b, v_{OH}), 2957/ 2921 (w, v_{C-H}), 1718 (m, v_{COOH}), 1637 (w, v_{C=C}), 1452 (w, δ_{P-C}), 1409, 1321, 1294, 1258, 1238 (w, v_{P-O}), 1151 (m, δ_{P-O-C}), 1006 (m, δ_{P-O-C}), 941, 813, 719, 651, 519, 456;
¹H-NMR (300 MHz, CDCl₃, δ[ppm]): 9,83 (s, a), 6,06 - 6,04 (m, 2H, l'), 5,56 - 5,52 (m, 2H, l"), 5,37 - 5,28 (m, 1H, h), 4,36 - 4,18 (m, 4H, i), 2,69 (t, ³J_{HH} = 6,80 Hz, 2H, f), 2,58 - 2,52 (q, 4H, d und e), 1,89 - 1,71 (m, 10H, b, c und m);
¹³C-NMR (75 MHz, CDCl₃, δ [ppm]): 171,14 (s, g), 166,83 (s, j), 135,56 (s, k), 126,59 (s, l), 69,46 (s, h), 62,47 (s, i), 34,66 (s, f), 32,31 (d, ³J_{PC} = 18,72 Hz, d), 26,54 (s, e), 24,67 (d, ¹J_{PC} = 144,55 Hz, b), 22,14 (d, ²J_{PC} = 3,64 Hz, c), 18,23 (s, m);
³¹P-NMR (121 MHz, CDCl₃, δ [ppm]): 35,33 (s, 1P);
MS (ESI) m/z: 439 [M + H⁺], 462 [M + Na⁺].

### Beispiel 6

### Schritt 1:

### Synthese von 1,3-Bis(4-(3-hydroxypropoxy)phenyl)prop-2-en-1-on

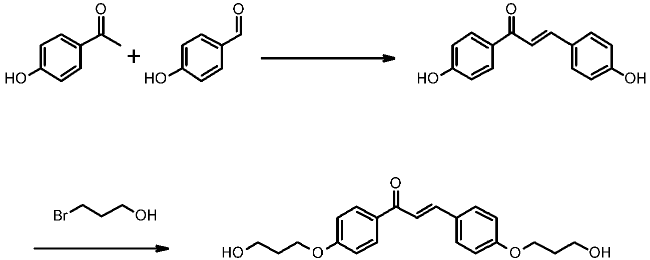

Das Chalkon 1,3-Bis(4-hydroxyphenyl)prop-2-en-1-on wurde durch eine literaturbekannte Methode über eine basenkatalysierte Claisen-Schmidt Kondensation von 4-Hydroxybenzaldehyd und 4-Hydroxyacetophenon hergestellt (vgl. N. Garg, T. Chandra, A. B. Jain and A. Kumar, European journal of medicinal chemistry 45:1529-1535 (2010)).

3,6 g (15 mmol) 1,3-Bis(4-hydroxyphenyl)prop-2-en-1-on und 4,2 g (30 mmol) Kaliumcarbonat wurden in 125 ml wasserfreiem Aceton gelöst. Nach Zugabe einer Spatelspitze Kaliumiodid wurden 3,4 ml (5,28 g, 38 mmol) 3-Brompropan-1-ol tropfenweise über einen Tropftrichter zugegeben. Die Mischung wurde ca. 23 Stunden unter Rückfluss erhitzt. Der Niederschlag wurde filtriert und mit Aceton gewaschen. Das klare, gelbe Filtrat wurde unter vermindertem Druck konzentriert und in Wasser gefällt. Der erhaltene Feststoff wurde in Aceton gelöst und nochmals mit Wasser ausgefällt. Nach Filtration wurde der gelbe Feststoff unter Vakuum getrocknet.

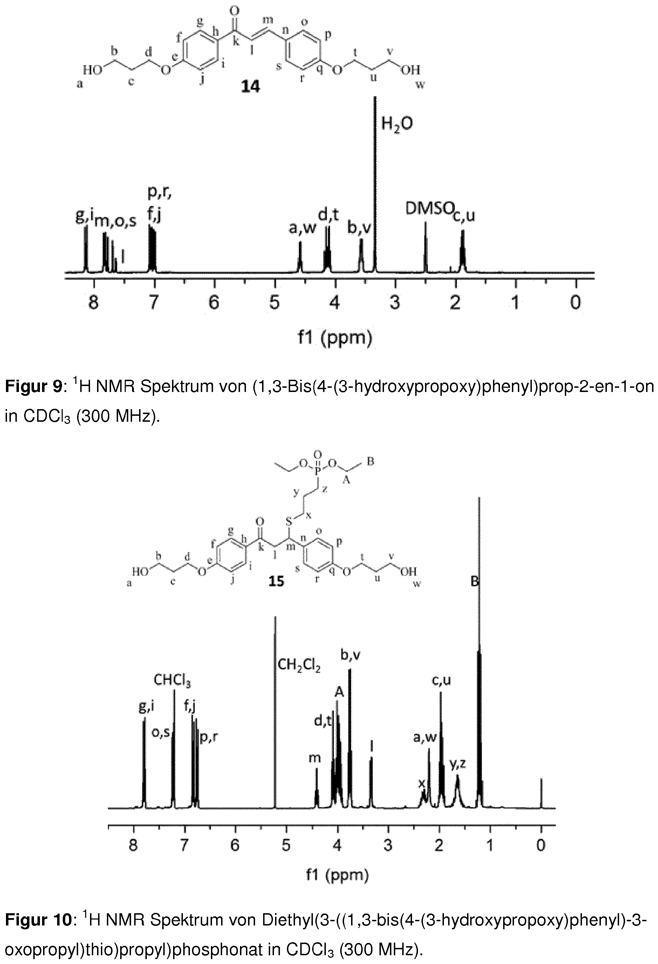

Das erhaltene ¹H NMR Spektrum von 1,3-Bis(4-(3-hydroxypropoxy)phenyl)prop-2-en-1-on ist in Figur 9 abgebildet.
Ausbeute: 37 % (5,5 mmol);
FT-IR (diamond, cm⁻¹): ṽ = 3269 (b, v_{OH}), 2952/ 2935/ 2874 (w, v_{C-H}), 1661, 1627 (m, v_{C=O}), 1600/ 1572/ 1509 (m, v_{Benzyl}), 1470, 1421, 1388, 1334, 1305, 1293, 1233, 1174 (s, v_{C-OH}), 1116, 1079, 1033, 992, 944, 875, 862, 845, 833, 817 (s, δ_{disubstituierter Ring}), 756, 670, 648, 621, 597, 554, 527, 517;
¹H-NMR (300 MHz, DMSO-d6, δ [ppm]): 8,15 (d, ³J_{HH} = 8,92 Hz, 2H, g und i), 7,85 - 7,78 (m, 3H, m, o und s), 7,68 (d, ³J_{HH} = 15,53 Hz, 1H, l), 7,08 (d, 2H, p und r), 7,01 (d, 2H, f und j), 4,59 (q, ³J_{HH} = 4,91 Hz, 2H, a und w), 4,18 - 4,09 (2t, ³J_{HH} = 6,47 Hz, 4H, d und t), 3,61 - 3,54 (m, 4H, b und v), 1,94 - 1,84 (m, 4H, c und u);
¹³C-NMR (75 MHz, DMSO-d6, δ [ppm]): 178,16 (s, k), 162,51 (s, e), 160,63 (s, q), 143,07 (s, m), 130,74 (s, g und i), 130,61 (s, o und s), 130,48 (s, h), 127,27 (s, n), 119,34 (s, l), 114,76 (s, f und j), 114,31 (s, p und r), 64,87 (d, J = 14,58 Hz, d und t), 57,14 (d, J = 3,51 Hz, b und v), 31,96 (d, J = 4,10 Hz, c und u);
MS (ESI) m/z: 357 [M + H⁺], 379 [M + K⁺].

### Schritt 2:

### Synthese von Diethyl(3-((1,3-bis(4-(3-hydroxypropoxy)phenyl)-3-oxopropyl)thio)-propyl)phosphonat

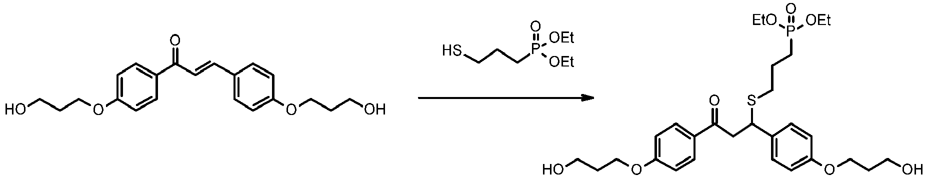

1g (2.8 mmol) 1,3-Bis(4-(3-hydroxypropoxy)phenyl)prop-2-en-1-on, 0,48 g (2.3 mmol) Diethyl(3-mercaptopropyl)phosphonat und 38,8 µl (28.1 mg, 0.3 mmol) Triethylamine wurden in 7 ml THF gelöst. Die Reaktionsmischung wurde durch Einleiten von Stickstoff über 20 Minuten gereinigt und 20 Stunden unter Rückfluss erhitzt. Nach einer Abkühlung der Reaktionsmischung auf Raumtemperatur wurden 30 ml Dichlormethan zugegeben und die Mischung wurde zweimal mit 1molarer, einmal mit 0,1molarer Salzsäure und mit Wasser gewaschen. Das organische Lösungsmittel wurde bei reduziertem Druck entfernt. Nicht umgesetztes Edukt wurde durch Säulenchromatographie (Silikagel) mit Ethylacetat abgetrennt. Das Produkt wurde durch Säulenchromatographie (Silikagel) mit Chloroform:Methanol (v:v 2:1) gereinigt.

Das erhaltene ¹H NMR Spektrum von Diethyl(3-((1,3-bis(4-(3-hydroxypropoxy)-phenyl)-3-oxopropyl)thio)propyl)phosphonat ist in Figur 10 abgebildet.
Ausbeute: 75 % (1,8 mmol);
R_{f}-Wert: 0,91 (Chloroform:Methanol 2:1);
FT-IR (diamond, cm⁻¹): ṽ = 3389 (b, v_{OH}), 2980/ 2932/ 2875 (w, v_{C-H}), 1737, 1672 (m, v_{C=O}), 1598/ 1574/ 1510 (m, v_{Benzyl}), 1472, 1421 (w, δ_{P-C}), 1392, 1334, 1304, 1248 (m, v_{P-O}), 1226, 1169 (s, v_{C-OH}), 1097, 1053, 1023 (s, δ_{P-O-C}), 956, 831 (m, δ_{disubstituierter Ring}), 734, 699, 623, 543;
¹H-NMR (300 MHz, CDCl₃, δ [ppm]): 7,80 (d, ³J_{HH} = 8,88 Hz, 2H, g und i), 7,23 (d, ³J_{HH}= 8,72 Hz, 2H, o und s), 6,83 (d, ³J_{HH} = 8,88 Hz, 2H, f und j), 6,76 (d, ³J_{HH}= 8,72 Hz, 2H, p und r), 4,41 (t, ³J_{HH} = 7,13 Hz, 1H, m), 4,09 (t, ³J_{HH} = 6,09 Hz, 4H, d und t), 4,06 - 3,90 (m, 4H, A), 3,79 -3,73 (2t, ³J_{HH} = 5,76 Hz, 4H, b und v), 3,35 (dd, ³J_{HH}= 7,08 Hz, 2H, l), 2,40 - 2,24 (m, 2H, x), 2,21 (s, 2H, a und w), 2,02 - 1,90 (m, 4H, c und u), 1,76 - 1,51 (m, 4H, y und z), 1,22 (t, ³J_{HH} = 7,06 Hz, 6H, B);
¹³C-NMR (75 MHz, CDCl₃, δ [ppm]): 195,49 (s, k), 162,95 (s, e), 157,95 (s, q), 134,08 (s, n), 130,41 (s, g und i), 129,82 (s, h), 128,87 (s, o und s), 114,49 (s, f und j), 114,21 (s, p und r), 65,47 (d, J = 3,56 Hz, d und t), 61,59 (d, J = 6,47 Hz, b und v), 59,76 (d, ²J_{PC} = 41,30 Hz, A), 45,02 (s, l), 44,01 (s, m), 32,02 (s, J = 9,39 Hz, c und u), 31,77 (s, x), 24,53 (d, ¹J_{PC} = 141,09 Hz, z), 22,19 (d, ²J_{PC} = 4,23 Hz, y), 16,43 (d, ³J_{PC} = 6,12 Hz, B);
³¹P-NMR (121 MHz, CDCl₃, δ [ppm]): 31,61 (s, 1P);
MS (ESI) m/z: 570 [M + H⁺]; EA_{calc.} (%): C 59,14, H 7,27, S 5,64; EA_{found} (%): C 59,28, H 7,20, S 5,29.

### Schritt 3:

### Synthese von methacrylatiertem Diethyl(3-((1,3-bis(4-(3-hydroxypropoxy)phenyl)-3-oxopropyl)thio)-propyl)phosphonat

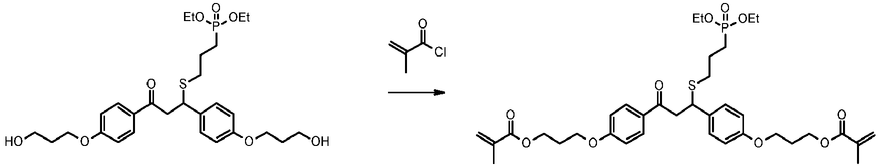

0,9 g (1.58 mmol) Diethyl(3-((1,3-bis(4-(3-hydroxypropoxy)phenyl)-3-oxopropyl)thio)-propyl)phosphonat und 0,5 ml (0.37 g, 3,66 mmol) Triethylamin wurden in 3,5 ml trockenem Dichlormethan gelöst und in einem Eisbad auf 0°C gekühlt. Nach einer Stunde wurden 0,36 ml (0,38 g, 3.64 mmol) Methacrylsäurechlorid, gelöst in 1 ml trockenem Dichlormethan, tropfenweise zugegeben. Die Lösung wurde auf Raumtemperatur gebracht und 20 Stunden gerührt. Gebildeter Niederschlag wurde filtriert und das Filtrat wurde dreimal mit 50 ml Wasser gewaschen. Das Lösungsmittel wurde unter vermindertem Druck entfernt. Das Rohprodukt wurde durch Säulenchromatographie (Silikagel) mit Ethylacetat gereinigt. Eine leicht gelbe Flüssigkeit mit honigartiger Konsistenz wurde erhalten.

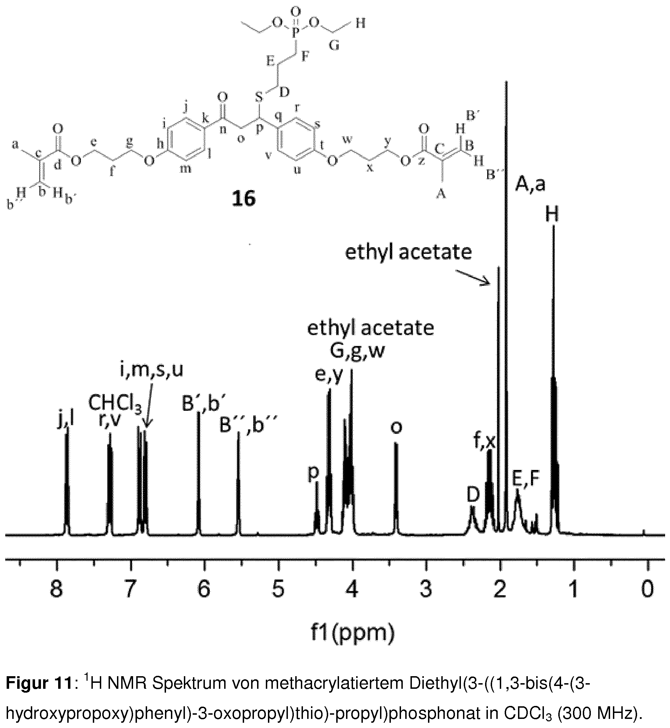

Das erhaltene ¹H NMR Spektrum von methacrylatiertem Diethyl(3-((1,3-bis(4-(3-hydroxypropoxy)phenyl)-3-oxopropyl)thio)-propyl)phosphonat ist in Figur 11 abgebildet.
Ausbeute: 81 % (1,3 mmol);
R_{f}-Wert: 0,47 (Ethylacetat);
FT-IR (diamond, cm⁻¹): ṽ = 2979/ 2930/ 2899/ 2872 (w, v_{C-H}), 1716 (m, v_{C=O}), 1676 (m, v_{C=C}), 1636, 1599/ 1575/ 1510 (m, v_{Benzyl}), 1472, 1452, 1421 (w, δ_{P-C}), 1319, 1296, 1232 (m, v_{P-O}), 1162 (s, v_{C-OH}), 1114, 1047, 1026 (s, δ_{P-O-C}), 954, 831 (m, δ_{disubstituted ring}), 814, 650, 624, 543;
¹H-NMR (300 MHz, CDCl₃, δ [ppm]): 7,81 (d, ³J_{HH} = 8,88 Hz, 2H, j und l), 7,24 (d, ³J_{HH} = 8,65 Hz, 2H, r und v), 6,83 (d, ³J_{HH} = 8,88 Hz, 2H, i und m), 6,75 (d, ³J_{HH}= 8,65 Hz, 2H, s und u), 6,04 - 6,02 (m, 2H, b' und B'), 5,50 - 5,47 (m, 2H, b" und B"), 4,43 (t, ³J_{HH} = 7,08 Hz, 1H; p), 4,26 (2t, ³J_{HH} = 6,10 Hz, 4H, e und y), 4,08 - 3,94 (m, 8H, g, w, G), 3,35 (d, ³J_{HH} = 7,08 Hz, 2H, o), 2,40 - 2,22 (m, 2H, D), 2,16 - 2,03 (m, 4H, f und x), 1,86 (s, 6H, a und A), 1,78 - 1,63 (m, 4H, E und F), 1,22 (t, ³J_{HH} = 7,02 Hz, 6H, H);
¹³C-NMR (75 MHz, CDCl₃, δ [ppm]): 195,29 (s, n), 167,32 (s, d), 167,28 (s, z), 162,77 (s, h), 157,85 (s, t), 136,26 (s, c), 136,19 (s, C), 134,11 (s, q), 130,39 (s, j und I), 129,95 (s, k), 128,88 (s, r und v), 125,62 (s, b), 125,51 (s, B), 114,42 (s, i und m), 114,17 (s, s und u), 64,69 (s, g), 64,33 (s, w), 61,55 (s,e), 61,46 (s, y), 61,38 (d, ²J_{PC} = 17,10 Hz, G), 45,08 (s, p), 43,75 (s, o), 31,90 (d, ³J_{PC} = 18,02 Hz, D), 28,67 (s, f), 28,51 (s, x), 24,65 (d, ¹J_{PC} = 141,03 Hz, F), 22,22 (d, ²J_{PC} = 4,24 Hz, E), 18,31 (s, a und A), 16,45 (d, ³J_{PC} = 5,81 Hz, H);
³¹P-NMR (121 MHz, CDCl₃, δ [ppm]): 31,46 (s, 1P);
MS (ESI) m/z: 705 [M + H⁺], EA_{calc.} (%): C 61,35, H 7,01, S 4,55; EA_{found} (%): C 60,65, H 6,94, S 4,63.

### Beispiel 7

### Synthese von (3-((1,3-Bis(4-3-8methacryloyloxy)propoxy)phenyl)-3-oxopropyl)thio)-propyl-phosphonsäure

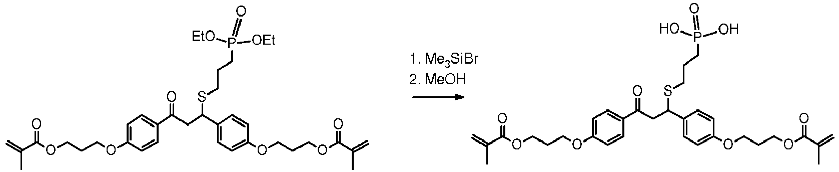

430 mg (0.61 mmol) methacrylatiertes Diethyl(3-((1,3-bis(4-(3-hydroxypropoxy)-phenyl)-3-oxopropyl)thio)-propyl)phosphonat wurden in einem 50 ml Zweihalsrundkolben in 6,2 ml trockenem Dichlormethan gelöst. Die Lösung wurde durch Einleiten von Stickstoff über 1 Stunde entgast. Anschließend wurden 805 µl (6,1 mmol) Bromtrimethylsilan zugegeben und die Lösung wurde bei einer Badtemperatur von 23°C für 9 Stunden gerührt. Das organische Lösungsmittel wurde bei vermindertem Druck entfernt und unter Vakuum getrocknet. Das Zwischenprodukt wurde mit 5 ml Methanol behandelt und 30 Minuten bei Raumtemperatur gerührt. Anschließend wurde das Lösungsmittel bei vermindertem Druck entfernt. Nach Trocknung des Produkts unter Vakuum wurde eine dunkelrote, hochviskose Flüssigkeit erhalten.

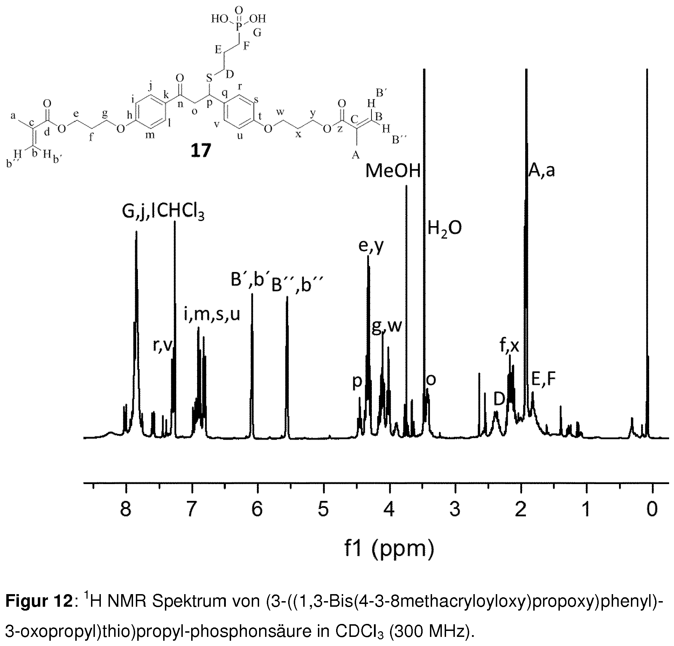

Das erhaltene ¹H NMR Spektrum von (3-((1,3-Bis(4-3-8methacryloyloxy)-propoxy)phenyl)-3-oxopropyl)thio)propyl-phosphonsäure ist in Figur 12 abgebildet.
Ausbeute: 89 % (0,54 mmol);
FT-IR (diamond, cm⁻¹): ṽ = 3417 (b, v_{O-H}), 2961/ 2924/ 2867 (w, v_{C-H}), 1714 (m, v_{C=O}), 1676 (m, v_{C=C}), 1635, 1598/ 1572/ 1510 (m, v_{Benzyl}), 1471, 1451, 1422 (w, δ_{P-C}), 1356, 1298, 1257 (s, v_{P-O}), 1166 (s, v_{C-OH}), 1112, 1043, 1011 (s, δ_{P-O-C}), 986, 827, 813 (s, δ_{disubstituted ring}), 734, 702, 648, 624, 621, 552, 543;
¹H-NMR (300 MHz, CDCl₃, δ [ppm]): 7,91 - 7,81 (m, 4H, j, l und G), 7,30 (d, ³J_{HH} = 8,59 Hz, 2H, r und v), 6,8 (d, ³J_{HH} = 8,93 Hz, 2H, i und m), 6,11 - 6,07 (m, 2H, s und u), 6,04 - 6,02 (m, 2H, b' und B'), 5,57 - 5,53 (m, 2H, b" und B"), 4,45 (t, ³J_{HH} = 7,03 Hz, 1H, p), 4,38 - 4,29 (m, 4H, e und y), 4,17 - 4,00 (m, 4H, g und w), 3,42 - 3,40 (m, 2H, o), 2,45 - 2,32 (m, 2H, D), 2,22 - 2,08 (m, 4H, f und x), 1,94 - 1,89 (m, 6H, a und A), 1,85 - 1,68 (m, 4H, E und F);
¹³C-NMR (75 MHz, CDCl₃, δ [ppm]): 196,91 (s, n), 167,65 (s, d), 167,51 (s, z), 163,25 (s, h), 158,03 (s, t), 136,30 (s, c), 136,26 (s, C), 133,89 (s, q), 130,38 (s, j und l), 129,74 (s, k), 128,99 (s, r und v), 125,87 (s, b), 125,84 (s, B), 114,69 (s, i und m), 114,45 (s, s und u), 64,91 (s, g), 64,54 (s, w), 61,76 (s, e), 61,46 (s, y), 45,04 (s, p), 43,55 (s, o), 31,53 (d, ³J_{PC} = 19,14 Hz, D), 28,74 (s, f), 28,60 (s, x), 24,55 (d, ¹J_{PC} = 141,63 Hz, F), 21,61 (d, ²J_{PC} = 2,80 Hz, E), 18,42 (s, a und A);
³¹P-NMR (121 MHz, CDCl₃, δ [ppm]): 35,46 (s, 1P);
MS (ESI) m/z: 581 [mono-methacryliertes M + H⁺]; 649 [M + H⁺].

## Patentansprüche

1. Härtbare Zusammensetzung zur Verwendung in der Dentalmedizin, umfassend mindestens ein vernetzendes Monomer mit mindestens einem Schwefelatom, darstellbar durch eine Struktur der Formel (I) 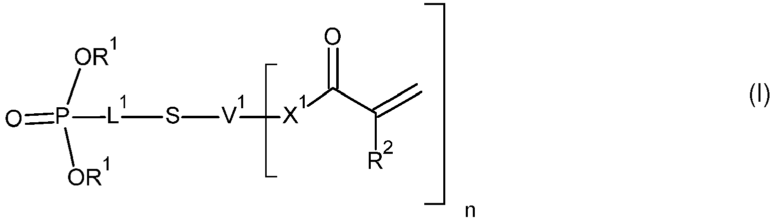 worin die Symbole folgende Bedeutung aufweisen:
L¹ ist eine lineare, verzweigte oder cyclische Alkylengruppe mit 2 bis 10 Kohlenstoffatomen ist, in der ein oder mehrere nicht benachbarte CH₂-Gruppen durch -R³C=CR³-, -C=C-, -(C=O)-, -(C=S)-, -(C=NR³)-, -C(=O)O-, -C(=O)NR³-, -NR³-, P(=O)(R³), -[CH(CₓH₂ₓ)P(=O)(OR¹)₂]-, -O-, -S-, -(SO)- oder (-SO₂)- ersetzt sein können, wobei x eine ganze Zahl im Bereich von 0 bis 6 ist, wobei das Phosphoratom der Gruppe -P=O(OR¹)₂ direkt mit einem Kohlestoffatom verbunden ist;
V¹ ist eine gesättigte oder ungesättigte, aliphatische oder heteroaliphatische Gruppe mit 2 bis 50 Kohlenstoffatomen; eine aromatische oder heteroaromatische Gruppe mit 5 bis 50 Kohlenstoffatomen oder eine Gruppe mit 6 bis 50 Kohlenstoffatomen, die mindestens einen gesättigten oder ungesättigten, aliphatischen und/oder heteroaliphatischen Rest und mindestens einen aromatischen und/oder heteroaromatischen Rest umfasst;
X¹ ist bei jedem Auftreten gleich oder verschieden O oder NR³;
R¹ ist H, Methyl, Ethyl, Propyl oder Butyl;
R² ist H oder ein Alkylrest mit 1 bis 4 Kohlenstoffatomen;
R³ ist H oder eine aliphatische, heteroaliphatische, aromatische oder heteroaromatische Gruppe mit 1 bis 6 Kohlenstoffatomen und
n ist 2, 3, 4, 5 oder 6,
**dadurch gekennzeichnet, dass** das Schwefelatom in der Struktur gemäß Formel (I) über höchstens 12 Bindungen von der Gruppe X¹ getrennt ist, die von dem Schwefelatom über die geringste Anzahl an Bindungen entfernt ist, wobei Doppelbindungen oder Dreifachbindungen als Einfachbindung gezählt werden.

2. Härtbare Zusammensetzung gemäß Anspruch 1, **dadurch gekennzeichnet, dass** die Gruppe V¹ mindestens einen aromatischen und/oder heteroaromatischen Rest aufweist, vorzugsweise mindestens einen aromatischen Rest.

3. Härtbare Zusammensetzung gemäß Anspruch 2, **dadurch gekennzeichnet, dass** der aromatische und/oder heteroaromatische Rest der Gruppe V¹ ein monocyclischer Rest ist, vorzugsweise ein Phenyl- oder Phenylen-Rest.

4. Härtbare Zusammensetzung gemäß einem der vorhergehenden Ansprüche, **dadurch gekennzeichnet, dass** das Symbol x in der Gruppe -[CH(CₓH₂ₓ)P(=O)(OR¹)₂]- eine ganze Zahl im Bereich von 1 bis 5 ist.

5. Härtbare Zusammensetzung gemäß einem der vorhergehenden Ansprüche, **dadurch gekennzeichnet, dass** die hydrolysierbare Gruppe R¹ ein Alkylrest mit 1 bis 4, vorzugsweise 2 Kohlenstoffatomen ist.

6. Härtbare Zusammensetzung gemäß einem der vorhergehenden Ansprüche, **dadurch gekennzeichnet, dass** das Monomer durch die Struktur (II) darstellbar ist 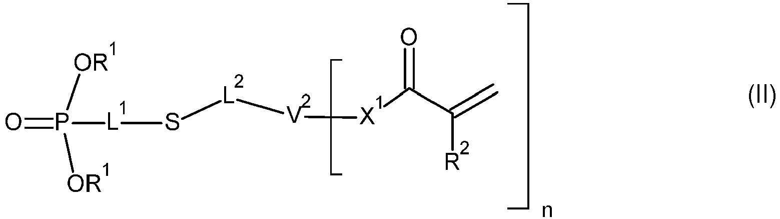 worin die Symbole n, L¹, X¹, R¹ und R² die in Anspruch 1 dargelegte Bedeutung aufweisen und
V² eine gesättigte oder ungesättigte, aliphatische und/oder heteroaliphatische Gruppe mit 2 bis 10, vorzugsweise 3 bis 6 Kohlenstoffatomen; eine aromatische und/oder heteroaromatische Gruppe mit 4 bis 12 Kohlenstoffatomen oder eine Gruppe mit 6 bis 14 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist; vorzugsweise ein Kohlenwasserstoffrest mit 3 bis 5 Kohlenstoffatomen; und
L² eine lineare, verzweigte oder cyclische, gesättigte oder ungesättigte, aliphatische und/oder heteroaliphatische Ether- oder Estergruppe mit 2 bis 30 Kohlenstoffatomen; eine aromatische und/oder heteroaromatische Ether- oder Estergruppe mit 5 bis 30 Kohlenstoffatomen oder eine Ether- oder Estergruppe mit 6 bis 30 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist.

7. Härtbare Zusammensetzung gemäß Anspruch 6, **dadurch gekennzeichnet, dass** die Gruppe V² ausgewählt ist aus folgenden Strukturen (V²-1), (V²-2) und/oder (V²-3) 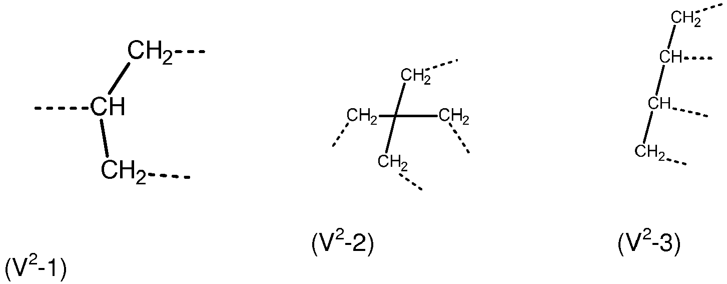 wobei die gestrichelten Linien die Bindungen an die Gruppen L² oder X¹ darstellen.

8. Härtbare Zusammensetzung gemäß Anspruch 6 oder 7, **dadurch gekennzeichnet, dass** die Gruppe L² durch eine Struktur der Formel (L²-1) darstellbar ist 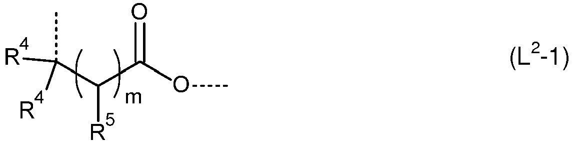 worin R⁴ bei jedem Auftreten H, eine Alkylgruppe mit 1 bis 10 Kohlenstoffatomen oder eine aromatische Gruppe mit 6 bis 8 Kohlenstoffatomen, vorzugsweise H oder eine Phenylgruppe ist; R⁵ bei jedem Auftreten unabhängig H oder eine Alkylgruppe mit 1 bis 10 Kohlenstoffatomen, vorzugsweise H ist; der Index m eine ganze Zahl im Bereich von 0 bis 8, vorzugsweise 0 bis 7 und besonders bevorzugt 0 bis 5 ist und die gestrichelten Linien jeweils die Bindung an das Schwefelatom oder die Gruppe V² darstellen.

9. Härtbare Zusammensetzung gemäß einem der vorhergehenden Ansprüche 1 bis 5, **dadurch gekennzeichnet, dass** das Monomer durch die Struktur (III) darstellbar ist 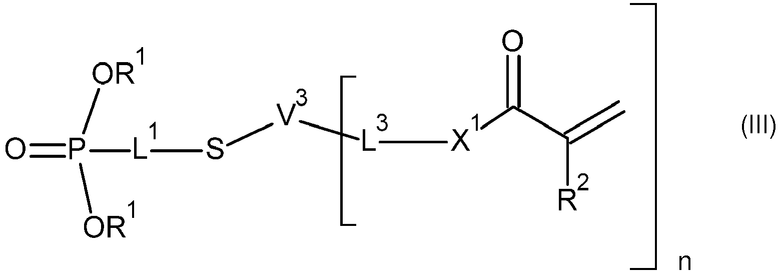 worin die Symbole n, L¹, X¹, R¹ und R² die in Anspruch 1 dargelegte Bedeutung aufweisen,
V³ eine gesättigte oder ungesättigte, aliphatische und/oder heteroaliphatische Gruppe mit 2 bis 10, vorzugsweise 3 bis 6 Kohlenstoffatomen; eine aromatische und/oder heteroaromatische Gruppe mit 4 bis 12 Kohlenstoffatomen oder eine Gruppe mit 6 bis 14 Kohlenstoffatomen ist, die mindestens einen gesättigten oder ungesättigten, aliphatischen und/oder heteroaliphatischen Rest und mindestens einen aromatischen und/oder heteroaromatischen Rest umfasst; vorzugsweise eine aliphatische Gruppe mit 2 bis 10, vorzugsweise 3 bis 6 Kohlenstoffatomen, in der ein oder mehrere nicht benachbarte CH₂-Gruppen durch -R³C=CR³-, -C≡C-, -(C=O)-, -(C=S)-, -(C=NR³),-, -C(=O)O-, -C(=O)NR³-, -NR³-, P(=O)(R³), -[CH(CₓH₂ₓ)P(=O)(OR¹)₂]-, -O-, -S-, -(SO)- oder (-SO₂)- ersetzt sein können, wobei R³ die in Anspruch 1 genannte Bedeutung aufweist und x eine ganze Zahl im Bereich von 0 bis 6, vorzugsweise 1 bis 5 ist; und
L³ eine lineare, verzweigte oder cyclische, gesättigte oder ungesättigte, aliphatische und/oder heteroaliphatische Ether- oder Estergruppe mit 6 bis 40, vorzugsweise 10 bis 25 Kohlenstoffatomen; eine aromatische und/oder heteroaromatische Ether- oder Estergruppe mit 6 bis 40, vorzugsweise 10 bis 25 Kohlenstoffatomen oder eine Ether- oder Estergruppe mit 6 bis 40, vorzugsweise 10 bis 25 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist; vorzugsweise eine aliphatische und/oder aromatische Ether- oder Estergruppe mit 6 bis 40, vorzugsweise 10 bis 25 Kohlenstoffatomen, in der ein oder mehrere nicht benachbarte CH₂-Gruppen durch -R³C=CR³-, -C≡C-, -(C=O)-, -(C=S)-, -(C=NR³),-, -C(=O)O-, -C(=O)NR³-, -NR³-, P(=O)(R³), -[CH(CₓH₂ₓ)P(=O)(OR¹)₂]-, -O-, -S-, -(SO)- oder (-SO₂)- ersetzt sein können, wobei R³ die in Anspruch 1 genannte Bedeutung aufweist und x eine ganze Zahl im Bereich von 0 bis 6, vorzugsweise 1 bis 5 ist.

10. Härtbare Zusammensetzung gemäß Anspruch 9, **dadurch gekennzeichnet, dass** die Gruppe L³ durch eine Struktur der Formel (L³-1) darstellbar ist 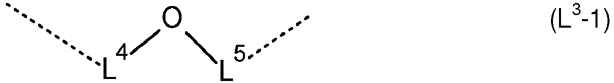 worin L⁴ und L⁵ bei jedem Auftreten unabhängig eine lineare, verzweigte oder cyclische Alkylengruppe mit 2 bis 20, vorzugsweise 3 bis 15 Kohlenstoffatomen ist, in der ein oder mehrere nicht benachbarte CH₂-Gruppen durch -R³C=CR³-, -C=C-, C=O, C=S, C=NR³, -C(=O)O-, -C(=O)NR³-, NR³, P(=O)(R³), -[CH(CₓH₂ₓ)P(=O)(OR¹)₂]-, -O-, -S-, SO oder SO₂ ersetzt sein können, wobei R¹ und R³ die in Anspruch 1 genannte Bedeutung haben und x eine ganze Zahl im Bereich von 0 bis 6, vorzugsweise 1 bis 5 ist, oder eine Aryl- oder Heteroaryl-Gruppe mit 5 bis 20 Kohlenstoffatomen, die durch ein oder mehrere Substituenten R³ substituiert sein kann; oder eine Gruppe mit 6 bis 40, vorzugsweise 10 bis 25 Kohlenstoffatomen, die mindestens einen gesättigten oder ungesättigten, aliphatischen und/oder heteroaliphatischen Rest und mindestens einen aromatischen und/oder heteroaromatischen Rest umfasst, ist; und die gestrichelten Linien jeweils die Bindung an die Gruppe V³ oder die Gruppe X¹ darstellen.

11. Härtbare Zusammensetzung gemäß Anspruch 10, **dadurch gekennzeichnet, dass** die Gruppe L⁴ durch eine Struktur der Formel (L⁴-1) oder (L⁴-2) darstellbar ist 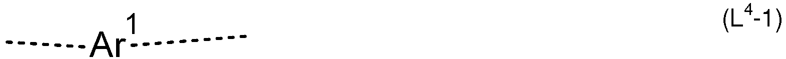 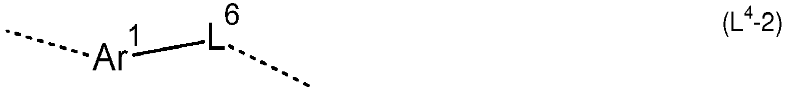 worin Ar¹ eine Aryl- oder Heteroaryl-Gruppe mit 4 bis 12 Kohlenstoffatomen, vorzugsweise 6 Kohlenstoffatomen ist, die durch ein oder mehrere Substituenten R³ substituiert sein kann; L⁶ ausgewählt ist aus C=O, C=S, C=NR³, -C(=O)O-, -C(=O)NR³-, NR³, P(=O)(R³), -O-, -S-, SO oder SO₂ oder eine lineare, verzweigte oder cyclische Alkylengruppe mit 1 bis 6 Kohlenstoffatomen, in der ein oder mehrere nicht benachbarte CH₂-Gruppen durch -R³C=CR³-, -C=C-, C=O, C=S, C=NR³, -C(=O)O-, -C(=O)NR³-, NR³, P(=O)(R³), -[CH(CₓH₂ₓ)P(=O)(OR¹)₂]-, -O-, -S-, SO oder SO₂ ersetzt sein können, wobei R³ die in Anspruch 1 genannte Bedeutung hat, x eine ganze Zahl im Bereich von 0 bis 6, vorzugsweise 1 bis 5 ist, und die gestrichelten Linien jeweils die Bindung an die Gruppe V³ oder das Sauerstoffatom darstellen.

12. Härtbare Zusammensetzung gemäß Anspruch 10 oder 11, **dadurch gekennzeichnet, dass** die Gruppe L⁵ durch eine Struktur der Formel (L⁵-1) darstellbar ist 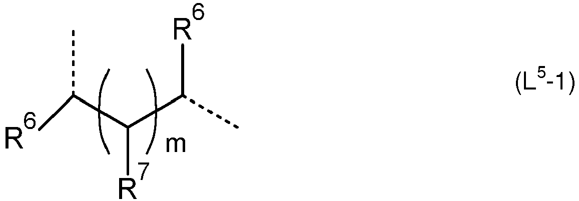 worin R⁶ bei jedem Auftreten unabhängig H, eine Alkylgruppe mit 1 bis 10 Kohlenstoffatomen oder eine aromatische Gruppe mit 6 bis 8 Kohlenstoffatomen, vorzugsweise H ist; R⁷ H oder eine Alkylgruppe mit 1 bis 10 Kohlenstoffatomen, vorzugsweise H ist; und der Index m eine ganze Zahl im Bereich von 0 bis 8, vorzugsweise 0 bis 7 und besonders bevorzugt 0 bis 5 ist und die gestrichelten Linien die Bindungen an das Sauerstoffatom oder die Gruppe X¹ darstellen.

13. Härtbare Zusammensetzung gemäß mindestens einem der Ansprüche 9, 10, 11 oder 12, **dadurch gekennzeichnet, dass** die Gruppe V³ durch eine Struktur der Formeln (V³-1), (V³-2), (V³-3), (V³-4), (V³-5) oder (V³-6) darstellbar ist 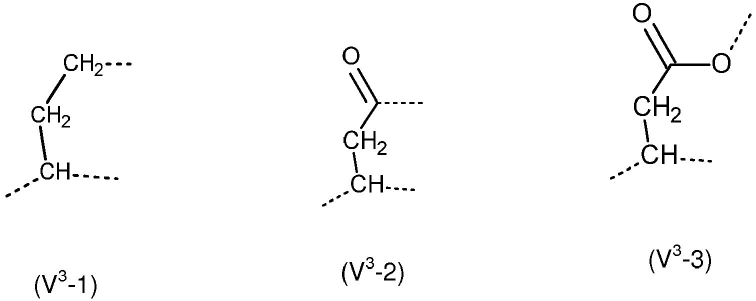 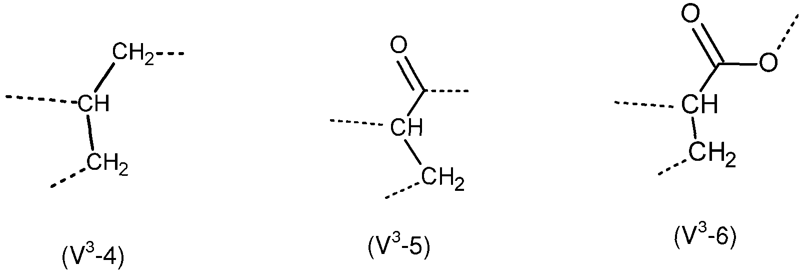 wobei die gestrichelten Linien die Bindungen an S oder die Gruppen L³ darstellen.

14. Härtbare Zusammensetzung gemäß mindestens einem der vorhergehenden Ansprüche zur Verwendung in der Dentalmedizin.

## Claims

1. A curable composition for use in dental medicine comprising at least one crosslinking monomer with at least one sulphur atom, representable by a structure of formula (I) 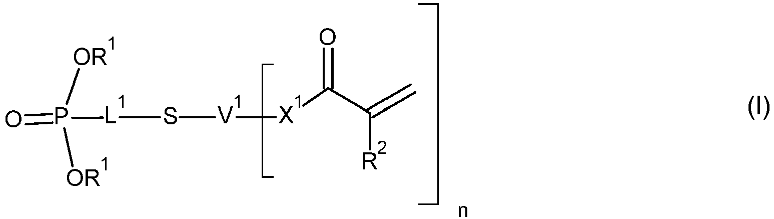 wherein the symbols have the following meaning:
L¹ is a linear, branched or cyclic alkylene group with 2 to 10 carbon atoms, in which one or more non-adjacent CH₂ groups can be substituted with -R³C=CR³-, -C≡C-, -(C=O)-, -(C=S)-, -(C=NR³)-, -C(=O)O-, -C(=O)NR³-, -NR³-, P(=O)(R³), -[CH(CₓH₂ₓ)P(=O)(OR¹)₂]-, -O-, -S-, -(SO)- or (-SO₂)-, wherein x is an integer in a range of 0 to 6, wherein the phosphorus atom of the group -P=O(OR¹)₂ is directly bonded with a carbon atom;
V¹ is a saturated or unsaturated, aliphatic or heteroaliphatic group with 2 to 50 carbon atoms; an aromatic or heteroaromatic group with 5 to 50 carbon atoms or a group with 6 to 50 carbon atoms, which comprises at least one saturated or unsaturated, aliphatic and/or heteroaliphatic residue and at least one aromatic and/or heteroaromatic residue;
X¹ is the same or different each time it occurs, either O or NR³;
R¹ is H, methyl, ethyl, propyl or butyl;
R² is H or an alkyl residue with 1 to 4 carbon atoms;
R³ is H or an aliphatic, heteroaliphatic, aromatic or heteroaromatic group with 1 to 6 carbon atoms, and
n is 2, 3, 4, 5 or 6,
**characterized in that**
the sulphur atom in the structure according to formula (I) is separated from group X¹ by a maximum of 12 bonds, which group is spaced from the sulphur atom by the lowest number of bonds, wherein double bonds or triple bonds are counted as a single bond.

2. The curable composition according to Claim 1, **characterized in that** group V¹ has at least one aromatic and/or heteroaromatic residue, preferably at least one aromatic residue.

3. The curable composition according to Claim 2, **characterized in that** the aromatic and/or heteroaromatic residue of group V¹ is a monocyclic residue, preferably a phenyl or phenylene residue.

4. The curable composition according to any one of the preceding claims, **characterized in that** the symbol x in the group -[CH(CₓH₂ₓ)P(=O)(OR¹)₂]- is an integer in a range of 1 to 5.

5. The curable composition according to any one of the preceding claims, **characterized in that** the hydrolysable group R¹ is an alkyl residue with 1 to 4, preferably 2, carbon atoms.

6. The curable composition according to any one of the preceding claims, **characterized in that** the monomer is representable by structure (II) 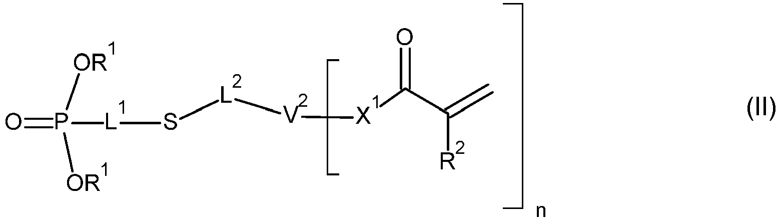 wherein the symbols n, L¹, X¹, R¹ and R² have the meaning described in Claim 1, and
V² is a saturated or unsaturated, aliphatic and/or heteroaliphatic group with 2 to 10, preferably 3 to 6, carbon atoms; an aromatic and/or heteroaromatic group with 4 to 12 carbon atoms or a group with 6 to 14 carbon atoms, which comprises at least one saturated or unsaturated, aliphatic and/or heteroaliphatic residue and at least one aromatic and/or heteroaromatic residue; preferably a hydrocarbon residue with 3 to 5 carbon atoms; and
L² is a linear, branched or cyclic, saturated or unsaturated, aliphatic and/or heteroaliphatic ether or ester group with 2 to 30 carbon atoms; an aromatic and/or heteroaromatic ether or ester group with 5 to 30 carbon atoms or an ether or ester group with 6 to 30 carbon atoms, which comprises at least one saturated or unsaturated, aliphatic and/or heteroaliphatic residue and at least one aromatic and/or heteroaromatic residue.

7. The curable composition according to Claim 6, **characterized in that** group V² is selected from the following structures (V²-1), (V²-2) and/or (V²-3) 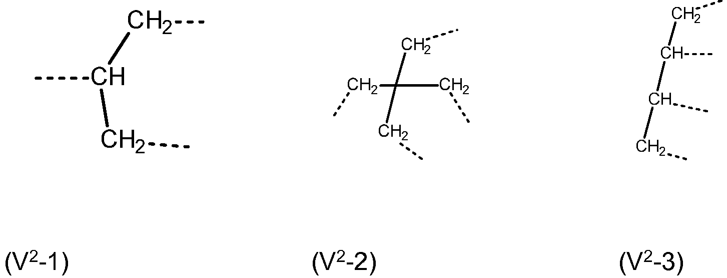 wherein the broken lines represent the bonds to groups L² or X¹.

8. The curable composition according to Claim 6 or 7, **characterized in that** group L² is representable by a structure of formula (L²-1) 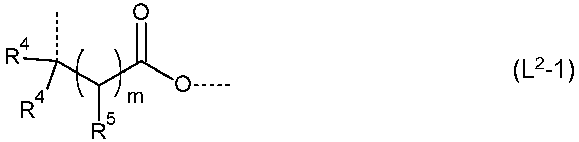 wherein, each time it occurs , R⁴ is H, an alkyl group with 1 to 10 carbon atoms or an aromatic group with 6 to 8 carbon atoms, preferably H or a phenyl group; each time it occurs, R⁵ is independently H or an alkyl group with 1 to 10 carbon atoms, preferably H; the index m is an integer in the range of 0 to 8, preferably 0 to 7, and particularly preferred 0 to 5, and the broken lines each represent the bond to the sulphur atom or group V².

9. The curable composition according to any one of the preceding Claims 1 to 5, **characterized in that** the monomer is also representable by structure (III) 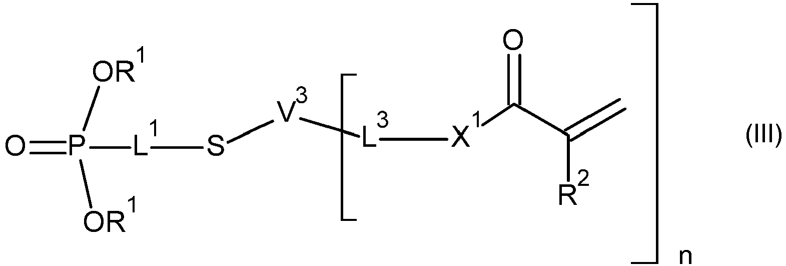 wherein the symbols n, L¹, X¹, R¹ and R² have the meaning described in Claim 1,
V³ is a saturated or unsaturated, aliphatic and/or heteroaliphatic group with 2 to 10, preferably 3 to 6, carbon atoms; an aromatic and/or heteroaromatic group with 4 to 12 carbon atoms or a group with 6 to 14 carbon atoms, which comprises at least one saturated or unsaturated, aliphatic and/or heteroaliphatic residue and at least one aromatic and/or heteroaromatic residue; preferably an aliphatic group with 2 to 10, preferably 3 to 6, carbon atoms, in which one or more non-adjacent CH₂-groups can be substituted with -R³C=CR³-, -C=C-, -(C=O)-, -(C=S)-, -(C=NR³),-, -C(=O)O-, -C(=O)NR³-, -NR³-, P(=O)(R³), -[CH(CₓH₂ₓ)P(=O)(OR¹)₂]-, -O-, -S-, -(SO)- or (-SO₂)-, wherein R³ has the meaning described in Claim 1 and x is an integer in the range of 0 to 6, preferably 1 to 5; and
L³ is a linear, branched or cyclic, saturated or unsaturated, aliphatic and/or heteroaliphatic ether or ester group with 6 to 40, preferably 10 to 25, carbon atoms; an aromatic and/or heteroaromatic ether or ester group with 6 to 40, preferably 10 to 25, carbon atoms or an ether or ester group with 6 to 40, preferably 10 to 25 carbon atoms, which comprises at least one saturated or unsaturated, aliphatic and/or heteroaliphatic residue and at least one aromatic and/or heteroaromatic residue; preferably an aliphatic and/or aromatic ether or ester group with 6 to 40, preferably 10 to 25, carbon atoms, in which one or more non-adjacent CH₂ groups can be substituted with -R³C=CR³-, -C=C-, -(C=O)-, -(C=S)-, -(C=NR³),-, -C(=O)O-, -C(=O)NR³-, -NR³-, P(=O)(R³), -[CH(CₓH₂ₓ)P(=O)(OR¹)₂]-, -O-, -S-, -(SO)- or (-SO₂)-, wherein R³ has the meaning described in Claim 1, and x is an integer in the range of 0 to 6, preferably 1 to 5.

10. The curable composition according to Claim 9, **characterized in that** group L³ is representable by a structure of formula (L³-1) 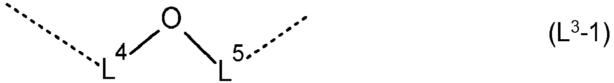 wherein, each time they occur, L⁴ and L⁵ independently are a linear, branched or cyclical alkylene group with 2 to 20, preferably 3 to 15, carbon atoms, in which one or more non-adjacent CH₂ groups can be substituted with
-R³C=CR³-, -C=C-, C=O, C=S, C=NR³, -C(=O)O-, -C(=O)NR³-, NR³, P(=O)(R³), -[CH(CₓH₂ₓ)P(=O)(OR¹)₂]-, -O-, -S-, SO or SO₂, wherein R¹ and R³
have the meaning described in Claim 1, and x is an integer in the range of 0 to 6, preferably 1 to 5, or an aryl or heteroaryl group with 5 to 20 carbon atoms, which can be substituted with one or more substituents R³; or a group with 6 to 40, preferably 10 to 25, carbon atoms, which comprises at least one saturated or unsaturated, aliphatic and/or heteroaliphatic residue and at least one aromatic and/or heteroaromatic residue; and the broken lines each represent the bond to group V³ or group X¹.

11. The curable composition according to Claim 10, **characterized in that** group L⁴ is representable by a structure of formula (L⁴-1) or (L⁴-2) 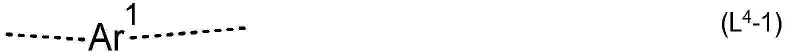 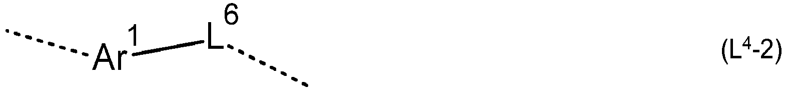 wherein Ar¹ is an aryl or heteroaryl group with 4 to 12 carbon atoms, preferably 6 carbon atoms, which can be substituted with one or more substituents R³; L⁶ is selected from C=O, C=S, C=NR³, -C(=O)O-, -C(=O)NR³-, NR³, P(=O)(R³), - O-, -S-, SO or SO₂ or a linear, branched or cyclic alkylene group with 1 to 6 carbon atoms, in which one or more non-adjacent CH₂-groups can be substituted with -R³C=CR³-, -C=C-, C=O, C=S, C=NR³, -C(=O)O-, -C(=O)NR³-, NR³, P(=O)(R³), -[CH(CₓH₂ₓ)P(=O)(OR¹)₂]-, -O-, -S-, SO or SO₂, wherein R³ has the meaning described in Claim 1, x is an integer in the range of 0 to 6, preferably 1 to 5, and the broken lines each represent the bond to group V³ or the oxygen atom.

12. The curable composition according to Claim 10 or 11, **characterized in that** group L⁵ is representable by a structure of formula (L⁵-1) 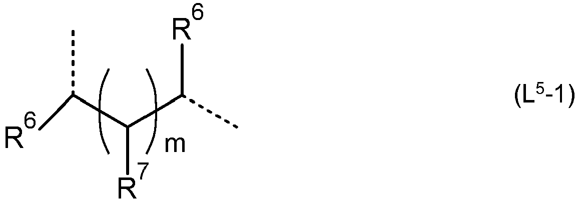 wherein, each time it occurs, R⁶ is independently H, an alkyl group with 1 to 10 carbon atoms or an aromatic group with 6 to 8 carbon atoms, preferably H; R⁷ is H or an alkyl group with 1 to 10 carbon atoms, preferably H; and the index m is an integer in the range of 0 to 8, preferably 0 to 7, and particularly preferred 0 to 5, and the broken lines represent the bonds to the oxygen atom or the group X¹.

13. The curable composition according to at least one of Claims 9, 10, 11 or 12, **characterized in that** group V³ is representable by a structure of formulae (V³-1), (V³-2), (V³-3), (V³-4), (V³-5) or (V³-6) 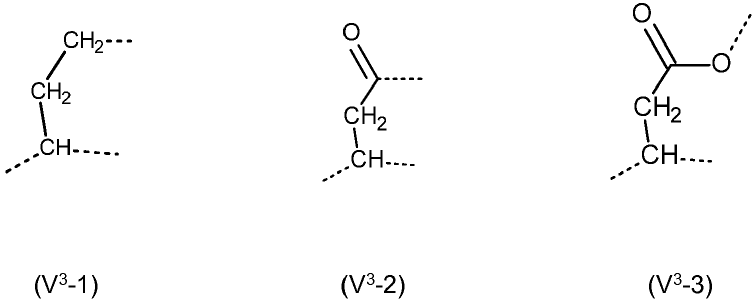 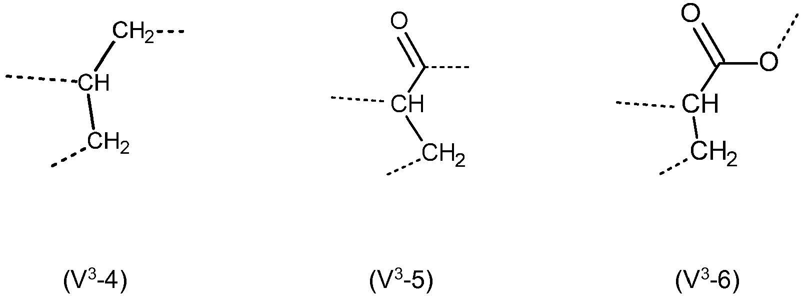 wherein the broken lines represent the bonds to S or groups L³.

14. The curable composition according to at least one of the preceding Claims for use in dental medicine.

## Revendications

1. Composition durcissable destinée à une utilisation en médecine dentaire, comprenant au moins un monomère de réticulation avec au moins un atome de soufre, pouvant être représenté par une structure selon la formule (I) 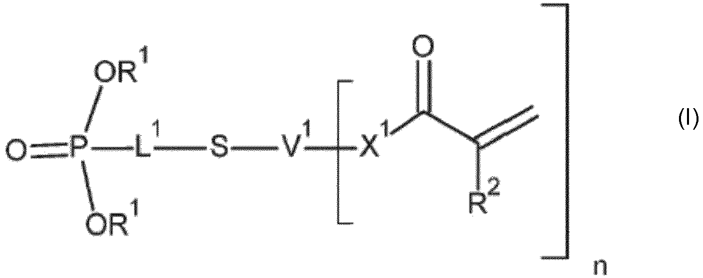 où les symboles présentent les significations suivantes :
L¹ est un groupe alcylène linéaire, ramifié ou cyclique avec de 2 à 10 atomes de carbone, dans lequel un ou plusieurs groupes CH₂- non voisins peuvent être remplacés par des groupes -R³C=CR³-, -C≡C-, -(C=O)-, -(C=S)-, -(C=NR³)-, -C(=O)O-, -C(=O)NR³-, -NR³-, P(=O)(R³), -[CH(CₓH₂ₓ)P(=O)(OR¹)₂]-, -O-, -S-, -(SO)- ou (-SO₂)-, où x est un nombre entier dans la plage de 0 à 6, où l'atome de phosphore du groupe -P=O(OR¹)₂ est relié directement avec un atome de carbone ;
V¹ est un groupe aliphatique ou hétéro aliphatique saturé ou insaturé avec de 2 à 50 atomes de carbone ; un groupe aromatique ou héréro aromatique avec de 5 à 50 atomes de carbone ou un groupe avec de 6 à 50 atomes de carbone qui comprend au moins un radical aliphatique et/ou hétéro aliphatique saturé ou insaturé et au moins un radical aromatique et/ou hétéro aromatique ;
X¹ est, en cas de présence, toujours identique ou différent de O ou de NR³ ;
R¹ est un atome H, un groupe méthyle, éthyle, propyle ou butyle ;
R² est un atome H ou un radical alkyle avec de 1 à 4 atomes de carbone ;
R³ est un atome H ou un groupe aliphatique, hétéro aliphatique, aromatique ou hétéro aromatique avec de 1 à 6 atomes de carbone, et
n est égal à 2, 3, 4, 5 ou 6,
**caractérisée en ce que**
l'atome de soufre dans la structure selon la formule (I) est séparé par au maximum 12 liaisons du groupe X¹, qui est éloigné de l'atome de soufre par le nombre minimal de liaisons, où les doubles liaisons ou les liaisons triples sont comptées comme des simples liaisons.

2. Composition durcissable selon la revendication 1, **caractérisée en ce que** le groupe V¹ présente au moins un radical aromatique et/ou hétéro aromatique, de préférence, au moins un radical aromatique.

3. Composition durcissable selon la revendication 2, **caractérisée en ce que** le radical aromatique ou hétéro aromatique du groupe V¹ est un radical monocyclique, de préférence un radical phényle ou phénylène.

4. Composition durcissable selon l'une des revendications précédentes, **caractérisée en ce que** le symbole x dans le groupe -[CH(CₓH₂ₓ)P(=O)(OR¹)₂]- est un nombre entier dans la plage de 1 à 5.

5. Composition durcissable selon l'une des revendications précédentes, **caractérisée en ce que** le groupe R¹ pouvant être hydrolysé est un radical alkyle avec de 1 à 4, de préférence avec 2 atomes de carbone.

6. Composition durcissable selon l'une des revendications précédentes, **caractérisée en ce que** le monomère peut être représenté par la structure (II) 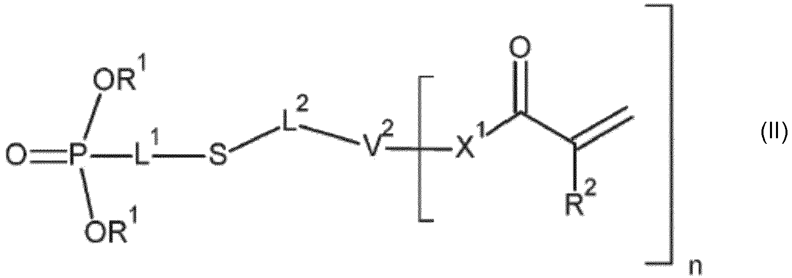 où les symboles n, L¹, X¹, R¹ et R² présentent les significations données en revendication 1, et
V² est un groupe aliphatique et/ou hétéro aliphatique saturé ou insaturé avec de 2 à 10, de préférence, de 3 à 6 atomes de carbone ; un groupe aromatique et/ou hétéro aromatique avec de 4 à 12 atomes de carbone ou un groupe avec de 6 à 14 atomes de carbone qui comprend au moins un radical aliphatique et/ou hétéro aliphatique saturé ou insaturé et au moins un radical aromatique et/ou hétéro aromatique ; de préférence, un radical hydrocarbure avec de 3 à 5 atomes de carbone ; et
L² est un groupe éther ou ester aliphatique et/ou hétéro aliphatique, linéaire, ramifié ou cyclique, saturé ou insaturé avec de 2 à 30 atomes de carbone ; un groupe éther ou ester aromatique et/ou hétéro aromatique avec de 5 à 30 atomes de carbone ou un groupe éther ou ester avec de 6 à 30 atomes de carbone, qui comprend au moins un radical aliphatique et/ou hétéro aliphatique saturé ou insaturé et au moins un radical aromatique et/ou hétéro aromatique.

7. Composition durcissable selon la revendication 6, **caractérisée en ce que** le groupe V² est choisi parmi les structures (V²-1), (V²-2), et/ou (V²-3) 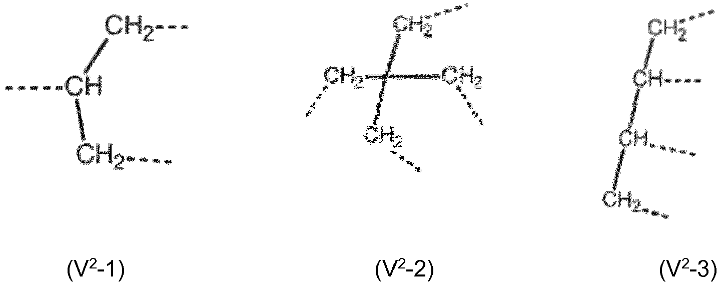 où les lignes en pointillés représentent les liaisons vers les groupes L² ou X¹.

8. Composition durcissable selon la revendication 6 ou la revendication 7, **caractérisée en ce que** le groupe L² peut être représenté par une structure de formule (L²-1) 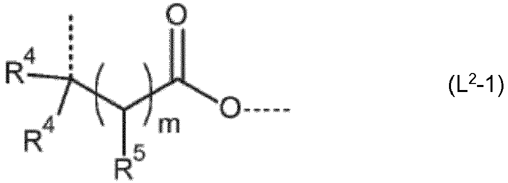 où R4, lors de chaque occurrence, est un groupe alkyle avec de 1 à 10 atomes de carbone ou un groupe aromatique avec de 6 à 8 atomes de carbone, est de préférence un atome H ou un groupe phényle ; R⁵, lors de chaque occurrence, est indépendamment un atome H ou un groupe alkyle avec de 1 à 10 atomes de carbone, est de préférence un atome H ; l'indice m est un nombre entier dans la plage de 0 à 8, de préférence de 0 à 7, et de manière particulièrement préférée de 0 à 5 et les lignes en pointillés représentent respectivement la liaison avec l'atome de soufre ou le groupe V².

9. Composition durcissable selon l'une des revendications précédentes 1 à 5, **caractérisée en ce que** le monomère peut être représenté par la structure (III) 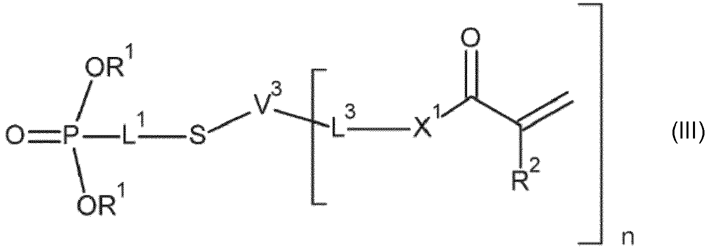 où les symboles n, L¹, X¹, R¹ et R² présentent les significations données en revendication 1, et
V³ est un groupe aliphatique et/ou hétéro aliphatique saturé ou insaturé avec de 2 à 10, de préférence, de 3 à 6 atomes de carbone ; un groupe aromatique et/ou hétéro aromatique avec de 4 à 12 atomes de carbone ou un groupe avec de 6 à 14 atomes de carbone qui comprend au moins un radical aliphatique et/ou hétéro aliphatique saturé ou insaturé et au moins un radical aromatique et/ou hétéro aromatique ; de préférence, un groupe aliphatique avec de 2 à 10, de préférence de 3 à 6 atomes de carbone dans lequel un ou plusieurs groupes CH₂- non voisins peuvent être remplacés par des groupes -R³C=CR³-, -C≡C-, -(C=O)-, -(C=S)-, -(C=NR³)-, -C(=O)O-, -C(=O)NR³-, -NR³-, P(=O)(R³), -[CH(CₓH₂ₓ)P(=O)(OR¹)₂]-, -O-, -S-, -(SO)- ou (-SO₂)-, où R³ présente ladite signification de la revendication 1 et x est un nombre entier dans la plage de 0 à 6, de préférence de 1 à 5 ; et
L³ est un groupe éther ou ester aliphatique et/ou hétéro aliphatique, linéaire, ramifié ou cyclique, saturé ou insaturé avec de 6 à 40 atomes de carbone, de préférence de 10 à 25 atomes de carbone ; un groupe éther ou ester aromatique et/ou hétéro aromatique avec de 6 à 40 atomes de carbone, de préférence de 10 à 25 atomes de carbone ou un groupe éther ou ester avec de 6 à 40, de préférence de 10 à 25 atomes de carbone, qui comprend au moins un radical aliphatique et/ou hétéro aliphatique saturé ou insaturé et au moins un radical aromatique et/ou hétéro aromatique ; de préférence un groupe éther ou ester avec de 6 à 40, de préférence de 10 à 25 atomes de carbone dans lequel un ou plusieurs groupes CH₂- non voisins peuvent être remplacés par des groupes -R³C=CR³-, -C≡C-, -(C=O)-, -(C=S)-, -(C=^{NR3})-, -C(=O)O-, -C(=O)NR³-, -NR³-, P(=O)(R³), -[CH(CₓH₂ₓ)P(=O)(OR¹)₂]-, -O-, -S-, -(SO)- ou (-SO₂)-, où R³ présente ladite signification de la revendication 1 et x est un nombre entier dans la plage de 0 à 6, de préférence de 1 à 5.

10. Composition durcissable selon la revendication 9, **caractérisée en ce que** le groupe L³ peut être représenté par une structure de formule (L³-1) 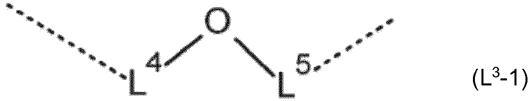 où L⁴ et L⁵, à chaque occurrence, sont indépendamment un groupe alkyle linéaire, ramifié ou cyclique avec de 2 à 20, de préférence de 3 à 15 atomes de carbone, dans lequel un ou plusieurs groupes CH₂- non voisins peuvent être remplacés par des groupes -R³C=CR³-, -C≡C-, -(C=O)-, -(C=S)-, -(C=NR³)-, -C(=O)O-, -C(=O)NR³-, -NR³-, P(=O)(R³), -[CH(CₓH₂ₓ)P(=O)(OR¹)₂]-, -O-, -S-, SO ou SO₂, où R¹ et R³ présentent ladite signification de la revendication 1 et x est un nombre entier dans la plage de 0 à 6, de préférence de 1 à 5, ou un groupe aryle ou hétéro aryle avec de 5 à 20 atomes de carbone qui peuvent être substitués par un ou plusieurs substituants R³ ; ou un groupe avec de 6 à 40, de préférence de 10 à 25 atomes de carbone qui comprend au moins un radical aliphatique et/ou hétéro aliphatique saturé ou insaturé et au moins un radical aromatique et/ou hétéro aromatique ; et les lignes en pointillés représentent respectivement la liaison au groupe V³ ou au groupe X¹.

11. Composition durcissable selon la revendication 10, **caractérisée en ce que** le groupe L⁴ peut être représenté par une structure de formule (L⁴-1) ou (L⁴-2) 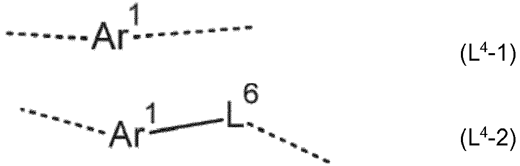 où Ar¹ est un groupe aryle ou hétéro aryle avec de 4 à 12 atomes de carbone, de préférence avec 6 atomes de carbone qui peut être substitué par un ou plusieurs substituants R³ ; L⁶ est choisi parmi les groupes C=O, C=S, C=NR³, -C(=O)O-, -C(=O)NR³, NR³, P(=O)(R³), -O-, -S-, SO ou SO₂ ou un groupe alcylène linéaire, ramifié ou cyclique avec de 1 à 6 atomes de carbone dans lequel un ou plusieurs groupes CH₂- non voisins peuvent être remplacés par des groupes -R³C=CR³-, -C≡C-, -(C=O)-, -(C=S)-, -(C=NR³)-, -C(=O)O-, -C(=O)NR³-, -NR³-, P(=O)(R³), -[CH(CₓH₂ₓ)P(=O)(OR¹)₂]-, -O-, -S-, SO ou SO₂, où R³ présente ladite signification de la revendication 1 et x est un nombre entier dans la plage de 0 à 6, de préférence de 1 à 5 , et les lignes en pointillés représentent respectivement la liaison au groupe V³ ou à l'atome d'oxygène.

12. Composition durcissable selon la revendication 10 ou la revendication 11, **caractérisée en ce que** le groupe L⁵ peut être représenté par une structure de formule (L⁵-1) 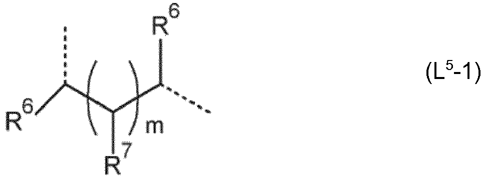 où R⁶, à chaque occurrence, est indépendamment un atome H, un groupe alkyle avec de 1 à 10 atomes de carbone ou un groupe aromatique avec de 6 à 8 atomes de carbone, est de préférence l'atome H ; R⁷ est un atome H ou un groupe alkyle avec de 1 à 10 atomes de carbone, est de préférence un atome H ; et l'indice m est un nombre entier dans la plage de 0 à 8, de préférence de 0 à 7 et de manière particulièrement préférée de 0 à 5 et les lignes en pointillés représentent les liaisons avec l'atome d'oxygène ou le groupe X¹.

13. Composition durcissable selon au moins l'une des revendications 9, 10, 11 ou 12, **caractérisée en ce que** le groupe V³ peut être représenté par une structure des formules (V³-1), (V³-2, (V³-3), (V³-4), (V³-5) ou (V³-6) 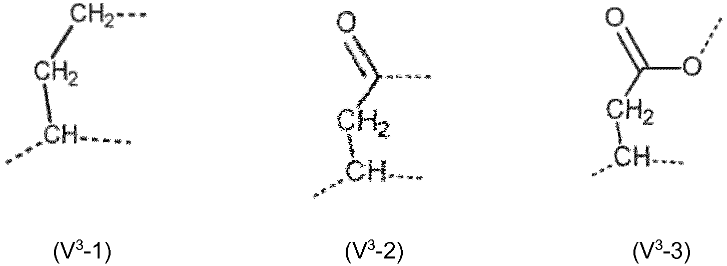 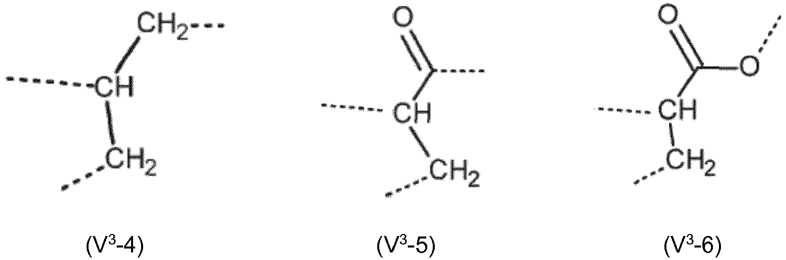 où les lignes en pointillés représentent les liaisons à l'atome S ou les groupes L³.

14. Composition durcissable selon au moins l'une des revendications précédentes pour l'emploi en médecine dentaire.
